(19) Europäisches Patentamt
European Patent Office
Office européen des brevets

(11) EP 0 897 920 A1

(12) EUROPEAN PATENT APPLICATION
published in accordance with Art. 158(3) EPC

(43) Date of publication:
24.02.1999 Bulletin 1999/08

(21) Application number: 97908555.2

(22) Date of filing: 31.03.1997

(51) Int. Cl.[6]: C07D 401/14, C07D 495/04
// A61K31/445, A61K31/495

(86) International application number:
PCT/JP97/01106

(87) International publication number:
WO 97/36887 (09.10.1997 Gazette 1997/43)

(84) Designated Contracting States:
BE DE ES FR GB IT

(30) Priority: 29.03.1996 JP 78004/96

(71) Applicant:
MEIJI SEIKA KABUSHIKI KAISHA
Tokyo 104 (JP)

(72) Inventors:
- YAMAMOTO, Takehiro,
Meiji Seika Kaisha, Ltd
Kouhoku-ku, Yokohama-shi, Kanagawa 222 (JP)
- MIURA, Tomoaki,
Meiji Seika Kaisha, Ltd
Kouhoku-ku, Yokohama-shi, Kanagawa 222 (JP)
- ISOMURA, Yasuko,
Meiji Seika Kaisha, Ltd
Kouhoku-ku, Yokohama-shi, Kanagawa 222 (JP)
- TADAUCHI, Kaori,
Meiji Seika Kaisha, Ltd
Kouhoku-ku, Yokohama-shi, Kanagawa 222 (JP)
- IIDA, Hiroyuki,
Meiji Seika Kaisha, Ltd
Kouhoku-ku, Yokohama-shi, Kanagawa 222 (JP)
- OUCHI, Shokichi,
Meiji Seika Kaisha, Ltd
Kouhoku-ku, Yokohama-shi, Kanagawa 222 (JP)
- KATANO, Kiyoaki,
Meiji Seika Kaisha, Ltd
Kouhoku-ku, Yokohama-shi, Kanagawa 222 (JP)

(74) Representative:
Hall, Marina et al
Elkington and Fife
Prospect House,
8 Pembroke Road
Sevenoaks, Kent TN13 1XR (GB)

(54) NOVEL HETEROCYCLIC COMPOUNDS HAVING PLATELET AGGREGATION INHIBITORY EFFECTS

(57) Disclosed are a compound represented by the following formula (I) and pharmacologically acceptable salt and solvate thereof having inhibitory activity against platelet aggregation:

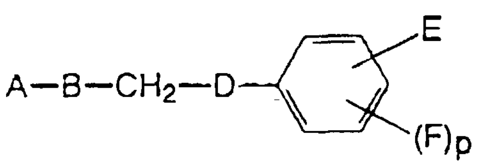

(I)

wherein A represents the following formula (II), (III), or (IV):



Printed by Xerox (UK) Business Services
2.16.7/3.6

(Cont. next page)

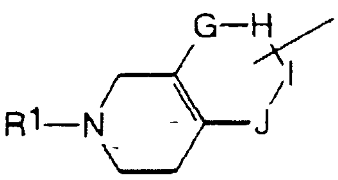

(II)

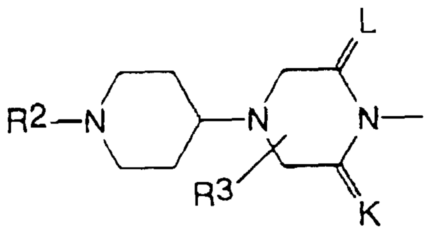

(III)

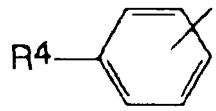

(IV)

A pharmaceutical composition comprising the above compound can be utilized as a platelet aggregation inhibitor that possesses excellent inhibitory activity against platelet aggregation, is free from hemorrhage and other side effects such as those derived from selectivity for the inhibitory action, and is potent against vasculature lesions, such as vasospasm.

## Description

[BACKGROUND OF THE INVENTION]

Field of the Invention

The present invention relates to compounds having inhibitory activity against platelet aggregation and a pharmaceutical composition, comprising at least one of these compounds as an active ingredient, that is useful for the treatment and prophylaxis of thrombotic diseases.

Background Art

Cardiovascular diseases are increased along with the change of dietary habits and the increase of advanced ages. Almost fifty percent of these diseases may he caused by thrombus.
Platelets in plasma are mainly associated with the formation of thrombus in organisms. For the purpose of the treatment and prophylaxis of thrombotic diseases in clinical practice, there have been used medicaments which suppress the functions of platelet and inhibits the aggregation of platelets, for example, aspirin which inhibits cyclooxygenase, ticlopidine which activates adenylcylase, and ozagrel which inhibit synthetase of thromboxane $A_2$.
In recent years, glycoproteins on platelet membrane have been progressively analyzed. As a result, it has been elucidated that the membrane glycoproptein called GPIIb/IIIa is a receptor of fibrinogen. This has therefore lead to the expectation that a GPIIb/IIIa antagonist would become an inhibitor of platelet aggregation having a novel action mechanism effectively used for the treatment and prophylaxis of the thrombotic diseases (Trends in Pharmacological Science, vol. 13, p.413 (1992)).
Compounds as the GPIIb/IIIa antagonist include, for example, monoclonal antibodies (Ann. New York Acad. Sci., vol. 614, p. 193 (1991)), tripeptide derivatives comprising arginine-glycine-aspartic acid (J. Med. Chem., vol. 35, p. 2040 (1992)); amidinophenyl derivatives (J. Med. Chem., vol. 35, p. 4393 (1992)); Japanese Patent Laid-Open Nos. 264068/1992 and 334351/1992, EP 483667, EP 502536, EP 525629, EP 529858, EP 537980, WO 9307867, WO 9402472 and the like), tyrosine derivatives (J. Med. Chem., vol. 35, p. 4640 (1992)), and piperidine derivatives (EP 512831, EP 540334, EP 578535 and the like). The present inventors also have found effective compounds in WO 9421599 and WO 9602503.
On the other hand, thromboxane $A_2$ ($TXA_2$) is one of metabolites of arachidonic acid and has physiological activities, such as platelet aggregation and vascular smooth muscle contraction activity. It is known that production of an excessive amount of $TXA_2$ within the body causes platelet aggregation activity, vascular occlusion, vasospasm, bronchoconstriction or the like, which is causative of the crisis of angina pectoris, cardiac infarction, cerebral infarction, bronchial asthma or the like. Therefore, an inhibitor against $TXA_2$ synthetase can be expected to be useful as a prophylactic or therapeutic agent for the angina pectoris, cardiac infarction, cerebral infarction, bronchial asthma and the like.
Conventional compounds having inhibitory activity against $TXA_2$ synthetase include, for example, compounds, such as ozagrel (The Merck Index, 11th edition, 6935) and Dazoxibene (Drug of the Future, vol. 6, 693 (1981), and pyridine derivatives (Japanese Patent Laid-Open Nos. 28963/1985, 100555/1985, 87570/1987, and 179425/1995).
Development of a medicament free from side effects, such as hemorrhage and having highly selective function as a therapeutic or prophylactic agent for thrombotic diseases has been desired in the art. Use of an GPIIb/IIIa antagonistic agent suffices for the inhibitory activity against platelet aggregation only. The GPIIb/IIIa antagonistic agent, however, is not potent against vascular lesions, such as vasospasm.

[SUMMARY OF THE INVENTION]

The present inventors have attempted to solve the above problem by combining the GPIIb/IIIa antagonism and inhibitory activity against $TXA_2$ synthetase.
The present inventors have made various studies and as a result have found compounds having a combination of GPIIb/IIIa antagonism with inhibitory activity against $TXA_2$ synthetase. Accordingly, an object of the present invention is to provide a novel compound having inhibitory activity against platelet aggregation.
Another object of the present invention is to provide a pharmaceutical composition comprising the novel compound having inhibitory activity against platelet aggregation.
Still another object of the present invention is to provide a method for the treatment or prophylaxis of thrombotic diseases, comprising the step of administering the novel compound having inhibitory activity against platelet aggregation.
A further object of the present invention is to provide use of the novel compound having inhibitory activity against platelet aggregation for the production of a pharmaceutical composition for the treatment or prophylaxis of

thrombotic diseases.

According to the present invention, there is provided an imidazole or pyridine derivative represented by the following formula (I) or a pharmacologically acceptable salt and a solvate thereof:

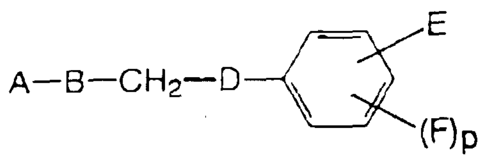

(I)

wherein

A represents the following formula (II), (III), or (IV):

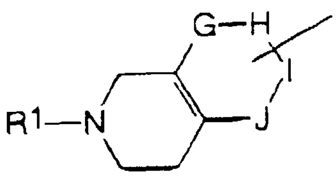

(II)

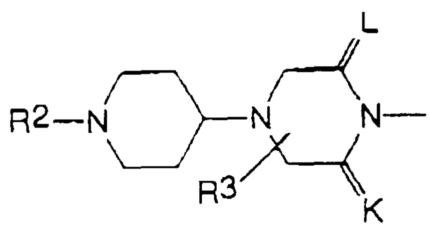

(III)

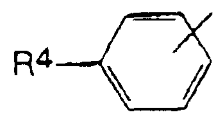

(IV)

wherein

$R^1$ and $R^2$, which may be the same or different, represents
a hydrogen atom or
lower alkyl in which one or more hydrogen atoms are optionally substituted by a hydroxyl group, a halogen atom, amino, or lower alkylamino;
$R^3$ represents
a hydrogen atom,
lower alkyl in which one or more hydrogen atoms are optionally substituted by a hydroxyl group, a halogen atom, amino, carboxyl, lower alkoxy, lower alkylamino, or lower alkoxycarbonyl,
phenyl in which one or more hydrogen atoms are optionally substituted by a hydroxyl group, a halogen atom, amino, carboxyl, lower alkoxy, lower alkylamino, lower alkoxycarbonyl, or halo lower alkyl, or
phenyl lower alkyl in which one or more hydrogen atoms are optionally substituted by a hydroxyl group, a halogen atom, amino, carboxyl, lower alkoxy, lower alkylamino, lower alkoxycarbonyl, or halo lower alkyl,
$R^4$ represents amidino or aminomethyl,
G, H, I, and J, which may be the same or different, represent $-CR^5=$, $-CR^5R^6-$, -N=, $-NR^5-$, -O-, -S-, -(CO)-, or a bond wherein $R^5$ and $R^6$, which may be the same or different, represent a hydrogen atom or lower alkyl, and
K and L represent $H_2$ or an oxygen atom;

B represents $-COCHR^7-$, $-CONR^7-$ wherein $R^7$ represents a hydrogen atom or lower alkyl, or a bond;
D represents -(CO)-, -CHOH-, or -CHY- wherein Y represents pyridyloxy, pyridyl, pyridyl lower alkyl, imidazolyl, or imidazolyl lower alkyl;
E represents a hydrogen atom (provided that E does not represent a hydrogen atom when D represents -(CO)-) or $-CH_2Y$ wherein Y is as defined above;
F represents group $-Z-(CH_2)_qCOOR^8$ or $-CH=CR^9COOR^8$ wherein

Z represents -O- or a bond,
$R^8$ represents a hydrogen atom, lower alkyl, or an ester residue removable under physiological conditions,
$R^9$ represents a hydrogen atom or lower alkyl, and
q is an integer of 1 to 4; and

p is 1 or 2.

The compound represented by the formula (I) according to the present invention can provide a platelet aggregation inhibitor that possesses excellent inhibitory activity against platelet aggregation, is free from hemorrhage and other side effects such as those derived from selectivity for the inhibitory action, and is potent against vasculature lesions, such as vasospasm.

[DETAILED DESCRIPTION OF THE INVENTION]

Compound of formula (I)

As used herein, the term "lower alkyl" as a group or a part of a group means straight-chain or branched alkyl having 1 to 6 carbon atoms, preferably 1 to 4 carbon atoms. The term "halogen atom" used herein means a fluorine, chlorine, bromine, or iodine atom. Further, the term "haloalkyl" means alkyl in which one or more hydrogen atoms are substituted by a halogen atom.

In the formulae (II) and (III), $R^1$ and $R^2$ represent a hydrogen atom or lower alkyl. One or more hydrogen atoms of the lower alkyl may be substituted, and specific examples of substituents usable herein include a hydroxyl group, a halogen atom (preferably a chlorine, bromine, or fluorine atom), amino, and lower alkylamino (preferably methylamino, ethylamino, propylamino, dimethylamino, or diethylamino).

In the formulae (II) and (III), $R^3$ represents a hydrogen atom, lower alkyl, phenyl, or phenyl lower alkyl. One or more hydrogen atoms of the lower alkyl may be substituted by a hydroxyl group, a halogen atom (preferably a chlorine, bromine, or fluorine atom), amino, carboxyl, lower alkoxy, lower alkylamino (preferably methylamino, ethylamino, propylamino, dimethylamino, or diethylamino), or lower alkoxycarbonyl. One or more hydrogen atoms of phenyl may be substituted by a hydroxyl group, a halogen atom (preferably a chlorine, bromine or fluorine atom), amino, carboxyl, lower alkoxy, lower alkylamino (preferably methylamino, ethylamino, propylamino, dimethylamino, or diethylamino), lower alkoxycarbonyl, or halo lower alkyl. Further, one or more hydrogen atoms of phenyl in the phenyl lower alkyl may be substituted by a hydroxyl group, a halogen atom (preferably a chlorine, bromine, or fluorine atom), amino, carboxyl, lower alkoxy, lower alkylamino (preferably methylamino, ethylamino, propylamino, dimethylamino, or diethylamino), lower alkoxycarbonyl, or halo lower alkyl.

In the formula (IV), $R^4$ represents amidino or aminomethyl.

Preferred examples of groups represented by the formula (II) include those wherein any one of G and J represents $-NR^5-$, -O-, or -S-, with the other representing a bond, and both H and I represent $-CR^5=$.

Specific examples of groups represented by the formula (II) include 4,5,6,7-tetrahydrothieno[3,2-c]pyridin-2-yl or 3-yl, 4,5,6,7-tetrahydrothieno[2,3-c]pyridin-2-yl or 3-yl, 1-methyl-4,5,6,7-tetrahydropyrrolo[3,2-c]pyridin-2-yl or 3-yl, 1-methyl-4,5,6,7-tetrahydropyrrolo[2, 3-c]pyridin-2-yl or 3-yl, 4,5,6,7-tetrahydrofuro[3,2-c]pyridin-2-yl or 3-yl, and 4,5,6,7-tetrahydrofuro[2, 3-c]pyridin-2-yl or 3-yl.

Preferred examples of groups represented by the formula (III) include those wherein any one of or both K and L represent an oxygen atom.

In the formula (I), group B represents group $-COCHR^7-$ or $-CONR^7-$. Preferred examples of lower alkyl represented by $R^7$ include methyl, ethyl, n-propyl, iso-propyl, and n-, iso-, sec-, or t-butyl.

Y in -CHY- represented by group D and Y in $-CH_2Y$ represented by group E each independently represent pyridyloxy, pyridyl, pyridyl lower alkyl, imidazolyl, or imidazolyl lower alkyl. Preferred examples of Y in -CHY- represented by D include pyridyloxy (preferably 3-pyridyloxy), pyridyl (preferably 3-pyridyl), pyridylmethyl (preferably 3-pyridylmethyl), and imidazolyl (preferably 1-imidazolyl). Preferred examples of Y in $-CH_2Y$ represented by group E include pyridyloxy (preferably 3-pyridyloxy) and imidazolyl (preferably 1-imidazolyl).

In the formula (I), group E represents a hydrogen atom or $-CH_2Y$. When group E represents a hydrogen atom, the compound, wherein group D represents -(CO)-, is excluded from the compounds of the present invention. Preferably, group E is bonded at the 2- or 3-position of phenyl.

In the formula (I), group F represents group $-Z-(CH_2)qCOOR^8$ or $-CH=CR^9COOR^8$. p represents the number of group F's. This means that when p = 1, one group F is present on phenyl, while when p = 2, two group F's are present on phenyl. Z represents an oxygen atom or a bond, preferably an oxygen atom. q is an integer of 1 to 4, preferably 1 or 2.

Preferred examples of lower alkyl represented by $R^8$ and $R^9$ include methyl, ethyl, n-propyl, iso-propyl, and n-, iso-, sec-, or t-butyl.

$R^8$ may represent an ester residue removable under physiological conditions, and specific examples thereof include pivaloyloxymethyl, 1-(cyclohexyloxycarbonyloxy)ethyl, and (5-methyl-2-oxo-1, 3-dioxol-4-yl)methyl.

Preferably, group F is bonded at 4-position of phenyl in the case of p = 1, while group F is bonded at the 3- and 4-positions of phenyl in the case of p = 2.

According to a preferred embodiment of the present invention, any one of D and E represents a group having Y.

A group of preferred compounds according to the present invention include the following group of compounds.

A first group of preferred compounds are such that A represents the formula (II) wherein B represents $-CONR^7-$ (preferably, $R^7$ represents a hydrogen atom or methyl). Among this group of compounds, a group of more preferred compounds are such that D represents -(CO)- or -CHY- wherein Y preferably represents pyridyloxy, pyridyl, pyridylmethyl, or imidazolyl. Another group of preferred compounds are such that A represents the formula (III) and B represents a bond. Among this group of compounds, a group of more preferred compounds are such that D represents -(CO)- or -CHY- wherein Y preferably represents imidazolyl.

The compound according to the present invention may be in the form of a salt. Salts usable herein include pharmacologically acceptable non-toxic salts. Preferred examples thereof include inorganic salts, such as sodium, potassium, magnesium, and calcium salts, acid addition salts, such as trifluoroacetate, hydrochloride, sulfate, oxalate, methanesulfonate, and citrate salts, and amino acid salts, such as glutamate and aspartate salts.

The compound according to the present invention may be in the form of a solvate. Preferred solvates usable herein include hydrate and ethanolate.

Preparation of compound of formula (I)

The compound according to the present invention may be synthesized by the following processes.

Protective groups for an amino group which are generally used in peptide synthesis may be used in the following processes. Specific examples of preferred protective groups usable herein include t-butoxycarbonyl, benzyloxycarbonyl, p-methoxybenzyloxycarbonyl, 2,2,2-trichloroethoxycarbonyl, trifluoroacetyl, allyloxycarbonyl, and trityl. Protective groups for a carboxyl group which are generally used in peptide synthesis may be used in the following processes. Specific examples of preferred protective groups usable herein include methyl, ethyl, t-butyl, benzyl, p-methoxybenzyl, p-nitrobenzyl, allyl, and benzhydryl.

Process (1)

Among the compounds represented by the formula (I), a compound wherein D represents -(CO)- may be synthesized by a method represented by the following scheme.

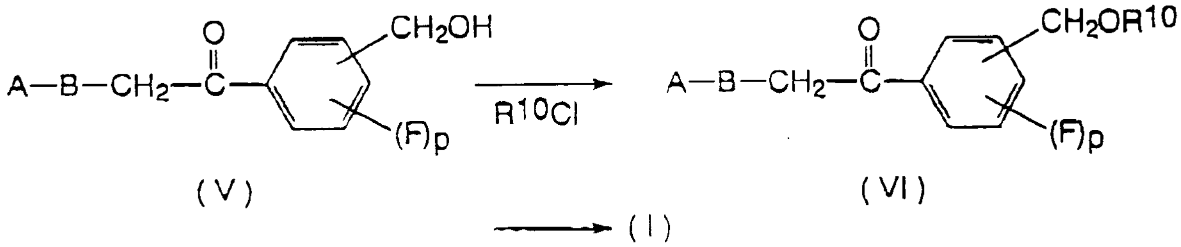


(V) (VI) (I)

wherein A, B, F, and p are as defined above in connection with the formula (I) with p being preferably 1; $R^1$, $R^2$, and $R^4$ are as defined above in connection with the formula (I) and, in addition, may represent a protective group for an amino group; $R^8$ is as defined above in connection with the formula (I) and, in addition, may represent a protective group for an ester group.

A compound represented by the formula (V) is reacted with a compound represented by the formula $R^{10}Cl$ (wherein $R^{10}$ represents an alkylsulfonyl, such as methanesulfonyl or p-toluenesulfonyl, or arylsulfonyl) in the presence of a base in a solvent not involved in the reaction (for example, methylene chloride or pyridine) for 0.5 to 24 hr, preferably 0.5 to 10 hr, at 0 to 100°C, preferably 0 to 30°C, to give a compound represented by the formula (VI).

The compound represented by the formula (VI) is then reacted with a compound capable of providing Y (specifically imidazole or pyridyl alcohol) in the presence of a base in a solvent not involved in the reaction (for example, acetone, tetrahydrofuran, N,N-dimethylacetamide or N,N-dimethylformamide) for 0.5 to 24 hr, preferably 1 to 10 hr, at 0 to 100°C, preferably 0 to 30°C, optionally followed by removal of a protective group. Thus, the compound represented by the formula (I) is prepared.

The compound represented by the formula (I) can be synthesized directly from the compound represented by the formula (V) by a method described in Synthesis, p. 1 (1981) (Mitsunobu's reaction). In this case, the compound represented by the formula (I) is obtained wherein group E represents group $-CH_2Y-$ wherein Y represents pyridyloxy (preferably 3-pyridyloxy).

The compound represented by the formula (I), wherein D represents -CHOH, may be prepared by reducing a compound represented by the formula (VI) according to a conventional method. In this case, the reduction may be carried out, for example, with sodium boron hydride.

The compound represented by the formula (V) may be synthesized, for example, from a compound, such as 4'-hydroxy-3'-methylacetophenone, according to a method described in WO 9421599 and WO 9602503.

Process (2)

The compound represented by the formula (I), wherein E represents a hydrogen atom, may be synthesized by a method represented by the following scheme:

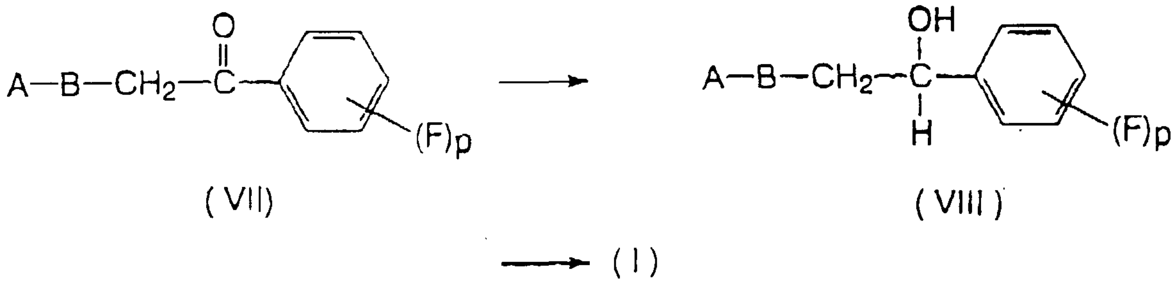

(VII) (VIII) (I)

wherein A, B, F, and p are as defined above in connection with the formula (I); $R^1$ and $R^4$ are as defined above in connection with the formula (I) and, in addition, may represent a protective group for an amino group; $R^8$ is as defined above in connection with the formula (I) and, in addition, may represent a protective group for an ester group.

At the outset, a compound represented by the formula (VII) is reduced according to a conventional method to give a compound represented by the formula (VIII). Next, the compound represented by the formula (VIII) is subjected to the above Mitsunobu's reaction to give the compound represented by the formula (I). More specifically, the Mitsunobu's reaction is carried out by reacting the compound represented by the formula (VIII) with a compound capable of providing Y (specifically imidazole or pyridyl alcohol) in a solvent not involved in the reaction (for example, tetrahydrofuran, diethyl ether, benzene, or 1,4-dioxane) in the copresence of triphenylphosphine and diethyl azodicarboxylate for 10 min to 24 hr, preferably 1 to 12 hr, at 0 to 100°C, preferably 20 to 80°C, optionally followed by removal of a protective group. Thus, the compound represented by the formula (I) is prepared.

The compound represented by the formula (I), wherein D represents group -CHY- wherein Y represents imidazolyl, can be prepared by subjecting the compound represented by the formula (VIII) to Mitsunobu's reaction using imidazole carboxylate or imidazole aldehyde as the compound capable of providing Y and optionally conducting deesterification or removal of the aldehyde.

Further, the compound represented by the formula (I) may be prepared using 2-amino-2-phenylethanol and 4'-hydroxyacetophenone as starting compounds according to a method described in EP 683156 or WO 9421599 and Chem. Pharm. Bull., 44, p.1510 (1996).

The compound represented by the formula (I), wherein D represents group -CHY- wherein Y represents pyridyl or pyridylalkyl, can be prepared using 4-bromophenol and 4-hydroxybenzyl cyanide as starting compounds according to a method described in Japanese Patent Laid-Open No. 43547/1993 and WO 9421599.

In the above processes, the sequence of the synthesis is determined so that a side reaction does not take place in the functional group not involved in the reaction. Further, it will be apparent to a person having ordinary skill in the art that the functional group may be protected by a suitable protective group so that an unfavorable reaction does not proceed.

Use of the compound of formula (I)/pharmaceutical composition

The compound according to the present invention inhibits the aggregation of platelets by inhibiting the binding between platelet membrane protein GPIIb/IIIa and fibrinogen. Further, the compound according to the present invention is expected to inhibit the production of $TXA_2$ by inhibition of $TXA_2$ synthetase and to inhibit release of. various cytokinins from platelets and twich of blood vessels. Thus, the compound according to the present invention and a pharmacologically acceptable salt thereof are effective in the treatment and prophylaxis of thrombotic disorders caused by the aggregation of platelets, particularly cerebral infarction, myocardial infarction, angina pectoris or peripheral arteriocclusion.

A pharmaceutical composition comprising the compound according to the present invention or a pharmacologically acceptable salt thereof as an active ingredient can be administered to human and non-human animal subjects through any one of route such as oral or parenteral routes such as intravenous injection, intramuscular injection, subcutaneous administration, rectal administration or percutaneous administration.

Therefore, the pharmaceutical composition comprising as an active ingredient the compound according to the present invention may be processed into suitable dosage forms depending on dosage routes, and can be specifically formed into preparations mainly including injections such as intravenous injection or intramuscular injection, oral preparations such as capsules, tablet, granule,powder, pill, grains or troche, rectal preparations, oily suppositories or aqueous suppositories.

These preparations can be prepared in the usual manners with conventional additives such as an excipient, a filler, a binder, a humidifier, a disintegrating agent, a surface active agent, a lubricant, a dispersant, a buffer, a preservative, a dissolution aid, an antiseptic agent, a flavoring agent, an analgesic agent or a stabilizer. The aforementioned acceptable and non-toxic additives include, for example,lactose, fructose, glucose, starch, gelatine, magnesium carbonate, synthetic magnesium silicate, talc, magnesium stearate, methyl cellulose or a salt thereof, gum arabic, polyethylene glycol, syrup, vaseline, glycerin, ethanol, propylene glycol, citric acid, sodium chloride, sodium sulfite and sodium phosphate.

The content of the compound according to the present invention in the pharmaceutical composition may vary depending on dosage forms. It, however, generally ranges from about 1 to 70% by weight, preferably from about 5 to 50% by weight of the total composition.

The dose is appropriately determined in consideration of the use, and the age, sex and severity of a patient. The dose is generally in the range from about 0.1 to 1,000 mg, preferably from 1 to 200 mg per day to an adult patient for the purpose of the treatment of thrombotic disorders. The dose may be administered in one or more portions per day.

[Examples]

Example 1

[2-(Imidazol-1-yl)methyl-4-[[[4,5,6,7-tetrahydrothieno[3,2-c]pyridin-2-yl]carbonylamino]acetyl]phenyl]oxyacetic acid trifluoroacetate (TFA)

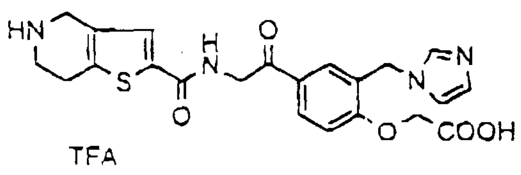

(a) 4'-Hydroxy-3'-methylacetophenone (450 mg) was dissolved in N,N-dimethylformamide (5 ml), potassium carbonate (435 mg) and t-butyl bromoacetate (0.46 ml) were added thereto, and the mixture was stirred at room temperature overnight. The reaction solution was diluted with ethyl acetate, washed with water, and dried over magnesium sulfate. The solvent was then removed under reduced pressure, and the residue was purified by column chromatography on silica gel (30 g, n-hexane : ethyl acetate = 3 : 1) to give 742 mg (yield: 94.0%) of t-butyl (4-acetyl-2-methylphenyl)oxyacetate.

$^1$H-NMR ($CDCl_3$) $\delta$: 1.48 (9H, s), 2.32 (3H, s), 2.55 (3H, s), 4.60 (2H, s), 6.69 (1H, d, J = 8.0 Hz), 7.77 (2H, m)
EIMS (m/z): 264 ($M^+$)

(b) The compound (10 g) prepared in the above stop (a) was dissolved in carbon tetrachloride (150 ml), N-bromosuccinimide (8.1 g) and 2,2'-azobis(isobutyronitrile) (50 mg) were added thereto, and the mixture was heated under reflux for 2 hr while irradiation with white light (200 W). After cooling, the insolubles were removed by filtration, and the filtrate was washed with an aqueous saturated sodium bicarbonate solution and then with water. The filtrate was then dried over magnesium sulfate, and the solvent was removed under reduced pressure to give t-butyl (4-acetyl-2-bromomethylphenyl)oxyacetate.

This product was dissolved without purification in N,N-dimethylformamide (70 ml). Potassium acetate (7.4 g)

was added thereto, and the mixture was stirred at room temperature overnight. Ethyl acetate was added thereto, the mixture was washed with water and dried over magnesium sulfate, and the solvent was removed under reduced pressure. The residue was purified by column chromatography on silica gel (300 g, n-hexane : ethyl acetate = 3 : 1) to give 6.02 g (49.3%) of t-butyl (4-acetyl-2-acetoxymethylphenyl)oxyacetate as an oil.

$^1$H-NMR ($CDCl_3$) δ: 1.47 (9H, s), 2.14 (3H, s), 2.57 (3H, s), 4.64 (2H, s), 5.25 (2H, s), 6.78 (1H, d, J = 8.6 Hz), 7.92 (1H, dd, J = 2.5, 8.6 Hz), 7.98 (1H, d, J = 2.5 Hz)
EIMS (m/z): 322 ($M^+$)

(c) The compound (6 g) prepared in the above step (b) was dissolved in 1,2-dichloroethane (40 ml), triethylamine (6.3 ml) was added to the solution, trimethylsilyl trifluoromethanesulfonate (4.54 ml) was added thereto under ice cooling, and the mixture was stirred at room temperature for one hr. The reaction solution was concentrated under reduced pressure, and dry ether was added to the residue, followed by stirring. The ether layer was obtained by decantation. The collection of the ether layer by decantation was repeated three times. The ether layers were combined, and the solvent was removed under reduced pressure. The residue was dissolved in tetrahydrofuran (60 ml), N-bromosuccinimide (3.36 g) was added thereto under ice cooling, and the mixture was stirred at room temperature for 2 hr. The reaction solution was concentrated, ethyl acetate was added to the concentrate, the mixture was washed with an aqueous saturated sodium bicarbonate solution, an aqueous sodium thiosulfate solution, and water in that order and then dried over magnesium sulfate, and the solvent was removed under reduced pressure. The residue was purified by column chromatography on silica gel (250 g, n-hexane : ethyl acetate = 5 : 1-4 : 1) to give t-butyl (2-acetoxymethyl-4-bromoacetylphenyl)oxyacetate as an oil.

The oil was dissolved in N,N-dimethylformamide (50 ml), sodium azide (1.21 g) was dissolved in the solution under ice cooling, and the mixture was stirred at room temperature for one hr. Ethyl acetate was added thereto, the mixture was washed with water and dried over magnesium sulfate, and the solvent was removed under reduced pressure. The residue was purified by column chromatography on silica gel (250 g, n-hexane : ethyl acetate = 4 : 1-2 : 1) to give 3.5 g (51.8%) of t-butyl (2-acetoxymethyl-4-azidoacetylphenyl)oxyacetate as an oil.

$^1$H-NMR ($CDCl_3$) δ: 1.47 (9H, s), 2.14 (3H, s), 4.52 (2H, s), 4.65 (2H, s), 5.25 (2H, s), 6.80 (1H, d, J = 8.6 Hz), 7.87 (1H, dd, J = 2.5, 8.6 Hz), 7.94 (1H, d, J = 2.5 Hz)
SIMS (m/z): 364 ($M^+$ + 1)

(d) The compound (3.5 g) prepared in the above step (c) was dissolved in a mixed solution composed of ethanol (70 ml) and 1 N ethanol hydrochloric acid (9.5 ml), 10%Pd-C (200 mg) was added to the solution, and catalytic reduction was carried out at room temperature under atmospheric pressure for 2.5 hr. The catalyst was filtered off through Celite, the filtrate was concentrated, and dichloromethane (20 ml) was added to the concentrate to prepare a solution of t-butyl (2-acetoxymethyl-4-aminoacetylphenyl)oxyacetate hydrochloride in dichloromethane. 5-t-Butoxycarbonyl-4,5,6,7-tetrahydrothieno[3,2-c]pyridin-2-carboxylic acid (2.7 g) was dissolved in dichloromethane (50 ml), benzotriazol-1-yloxy-tris(dimethylamino)phosphonium hexafluorophosphate (BOP) (4.20 g) was added to the solution, and the mixture was stirred at room temperature for one hr. The solution of t-butyl (2-acetoxymethyl-4-aminoacetylphenyl)oxyacetate hydrochloride in dichloromethane prepared above and diisopropylethylamine (3.5 ml) were added thereto. A reaction was allowed to react at room temperature for 2 hr. Ethyl acetate was added thereto, and the mixture was washed with water, dilute hydrochloric acid, water, an aqueous saturated sodium bicarbonate solution, and water in that order and then dried over magnesium sulfate. The solvent was removed under reduced pressure, and the residue was purified by column chromatography on silica gel (250 g, chloroform : ethyl acetate = 4 : 1) to give 5.06 g (92.0%) of t-butyl [2-acetoxymethyl-4-[[(5-t-butoxycarbonyl-4,5,6,7-tetrahydrothieno[3,2-c]pyridin-2-yl)carbonylamino]acetyl]phenyl]oxyacetate as an oil.

$^1$H-NMR ($CDCl_3$) δ: 1.48 (9H, s), 1.50 (9H, s), 2.16 (3H, s), 2.89 (2H, brs), 3.74 (2H, brs), 4.51 (2H, brs), 4.66 (2H, s), 4.87 (2H, d, J = 3.8 Hz), 5.26 (2H, s), 6.82 (1H, d, J = 8.7 Hz), 7.34 (1H, s), 7.97 (1H, dd, J = 2.3, 8.7 Hz), 8.04 (1H, d, J = 2.3 Hz)
FDMS (m/z): 602 ($M^+$)

(e) The compound (4.8 g) prepared in the above step (d) was dissolved in a mixed solution composed of methanol (100 ml) and tetrahydrofuran (20 ml), a magnesium chip (204 mg) was added thereto, and the mixture was stirred at room temperature for 2 hr. The reaction solution was then concentrated under reduced pressure, and ethyl acetate and water were added thereto. Insolubles were filtered off from the reaction solution through Celite, and the ethyl acetate layer was washed with water, dried over magnesium sulfate, and then concentrated under reduced pressure. The residue was purified by column chromatography on silica gel (250 g, chloroform : ethyl acetate = 2 :

1) to give 2.21 g (49.4%) of t-butyl [2-hydroxymethyl-4-[[(5-t-butoxycarbonyl-4,5,6,7-tetrahydrothieno[3,2-c]pyridin-2-yl)carbonylamino]acetyl]phenyl]oxyacetate as an oil.

$^1$H-NMR ($CDCl_3$) δ: 1.49 (18H, brs), 2.87 (2H, brs), 3.06 (1H, t, J = 6.5 Hz), 3.74 (2H, brs), 4.50 (2H, brs), 4.68 (2H, s), 4.79 (2H, d, J = 6.5 Hz), 4.87 (2H, d, J = 4.1 Hz), 6.86 (1H, d, J = 8.8 Hz), 7.03 (1H, t, J = 4.1 Hz), 7.33 (1H, s), 7.96 (1H, dd, J = 2.3, 8.8 Hz), 8.02 (1H, d, J = 2.3 Hz)
FDMS (m/z): 521 ($M^+$)

(f) The compound (116 mg) prepared in the above step (e) was dissolved in methylene chloride (2 ml), methanesulfonyl chloride (28.4 mg) dissolved in methylene chloride (0.5 ml) and diisopropylethylamine (34.8 mg) were added dropwise under ice cooling to the solution, and the mixture was stirred at room temperature for 2 hr. The reaction solution was diluted with ethyl acetate, washed with 0.1 N hydrochloric acid, an aqueous saturated sodium bicarbonate solution, and saturated saline, and then dried over magnesium sulfate. The solvent was removed under reduced pressure to give 122 mg (92.4%) of t-butyl[2-methanesulfonyloxymethyl-4-[[(5-t-butoxycarbonyl-4,5,6,7-tetrahydrothieno[3,2-c]pyridin-2-yl)carbonylamino]acetyl]phenyl]oxyacetate.

$^1$H-NMR ($CDCl_3$) δ: 1.49 (9H, s), 1.50 (9H, s)2.88 (2H, brs), 3.08 (3H, s), 3.74 (2H, brs), 4.51 (2H, brs), 4.68 (2H, s), 4.87 (2H, d, J = 4.0 Hz), 5.38 (2H, s), 6.87 (1H, d, J = 9.0 Hz), 6.99 (1H, m), 7.34 (1H, s), 8.04 (1H, dd, J = 2.0, 9.0 Hz), 8.10 (1H, d, J = 2.0 Hz)
SIMS (m/z): 639 ($M^+$ + 1)

(g) The compound (120 mg) prepared in the above step (f) was dissolved in acetone, imidazole(15 mg) and diisopropylethylamine (12 mg) were added to the solution, and the mixture was stirred at room temperature for one hr. The reaction solution was diluted with ethyl acetate, washed twice with saturated saline and dried over magnesium sulfate, and the solvent was removed under reduced pressure. The residue was purified by column chromatography on silica gel (12 g, chloroform : methanol = 50 : 1) to give 28 mg (24.5%) of t-butyl [2-(imidazol-1-yl)methyl-4-[[(5-t-butoxycarbonyl-4,5,6,7-tetrahydrothieno[3,2-c]pyridin-2-yl)carbonylamino]acetyl]phenyl]oxyacetate.

$^1$H-NMR ($CDCl_3$) δ: 1.49 (9H, s), 1.50 (9H, s), 2.87 (2H, brs), 3.73 (2H, brs), 4.50 (2H, brs), 4.65 (2H, s), 4.83 (2H, 8, J = 4.0 Hz), 5.22 (2H, s), 6.84 (1H, d, J = 9.0 Hz), 6.97 (1H, m), 7.04 (2H, m), 7.32 (1H, s), 7.67 (1H, s), 7.83 (1H, d, J = 2.0 Hz), 7.97 (1H, dd, J = 2.0, 9.0 Hz)
EIMS (m/z): 610 ($M^+$)

(h) The compound (25 mg) prepared in the above step (g) was dissolved in methylene chloride (0.1 ml), and anisole (0.01 ml) was added to the solution. Trifluoroacetlc acid (0.1 ml) was added to the mixture under ice cooling, and the mixture was stirred at room temperature for 30 mln. Isopropyl ether was added to the reaction solution, and the precipitated crystal was collected by filtration. This crystal was then dissolved in water and then lyophilized to give 12 mg (52.2%) of the title compound.

$^1$H-NMR ($D_2O$) δ: 3.28 (2H, t, J = 6.0 Hz), 3.66 (2H, t, J = 6.0 Hz), 4.41 (1H, s), 4.87 (2H, s), 4.94 (2H, s), 5.58 (2H, s), 7.15 (1H, d, J = 9.0 Hz), 7.46 (1H, m), 7.58 (1H, m), 7.60 (1H, s), 8.18 (1H, dd, J = 2.0, 9.0 Hz), 8.21 (1H, d, J = 2.0 Hz), 8.93 (1H, s)
SIMS (m/z): 455 ($M^+$ + 1)

Example 2

[2-(Pyridin-3-yl)oxymethyl-4-[[[4,5,6,7-tetrahydrothieno[3,2-c]pyridin-2-yl]carbonylamino]acetyl]phenyl]oxyacetic acid trifluoroacetate

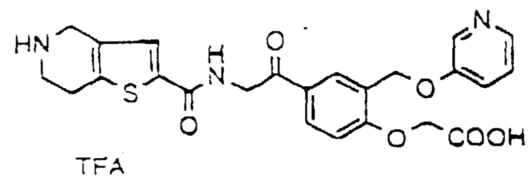

(a) The compound (262.6 mg) prepared in Example 1 (f) was dissolved in N,N-dimethylformamide (2 ml), 3-hydroxypyridine (47 mg) and diisopropylethylamine (0.143 ml) were added to the solution, and the mixture was stirred at room temperature overnight. Saturated saline was added to the reaction solution, and the mixture was extracted with ethyl acetate. The organic layer wad dried over magnesium sulfate, and the solvent was removed under reduced pressure. The residue was purified by column chromatography on silica gel (8 g, chloroform : methanol = 8 : 1) to give 35.3 mg (13.5%) of t-butyl [2-(pyridin-3-yl)oxymethyl-4-[[(5-t-butoxycarbonyl-4,5,6,7-tetrahydrothieno[3,2-c]pyridin-2-yl)carbonylamino]acetyl]phenyl]oxyacetate.

$^1$H-NMR ($CDCl_3$) δ: 1.49 (18H, s), 2.84 (2H, br s), 3.75 (2H, brs), 4.45 (2H, s), 4.64 (2H, s), 4.83 (2H, d, J = 1.5 Hz), 5.39 (2H, s), 6.87 (1H, d, J = 2.9 Hz), 7.21 (1H, d, J = 1.8 Hz), 7.38 (1H, brs), 7.51 (1H, brs), 7.75 (2H, brs), 8.02 (1H, d, J = 2.9 Hz), 8.21 (1H, s)
FDMS (m/z): 637 ($M^+$)

(b) The compound (47.2 mg) prepared in the above step (a) was dissolved in methylene chloride (0.2 ml), and anisole (0.02 ml) was added thereto. Trifluoroacetic acid (0.2 ml) was added to this solution under ice cooling, and the mixture was stirred for 30 min and then stirred at room temperature for additional one hr. The solvent was removed under reduced pressure, and the residue was dissolved in water. The solution was washed with isopropyl ether and lyophilized to give 37.9 mg (86.0%) of the title compound.

$^1$H-NMR ($D_2O$) δ: 3.19 (2H, brs), 3.60 (2H, brs), 4.34 (2H, brs), 4.88 (2H, s), 5.77 (2H, s), 7.01 (1H, m), 7.10 (2H, d, J = 3.0 Hz), 7.53 (1H, s), 7.80 (1H, m), 7.90 (1H, d, J = 2.1 Hz), 8.13 (1H, d, J = 3.0 Hz), 8.25 (1H, s), 8.47 (1H, brs)
FDMS (m/z): 482 ($M^+$ + 1)

Example 3

[[4-[2-[[4,5,6,7-Tetrahydrothieno[3,2-c]pyridin-2-yl]carbonyl]amino-1-[(pyridin-3-yl)oxy]ethyl]-o-phenylene]dioxy]diacetic acid trifluoroacetate

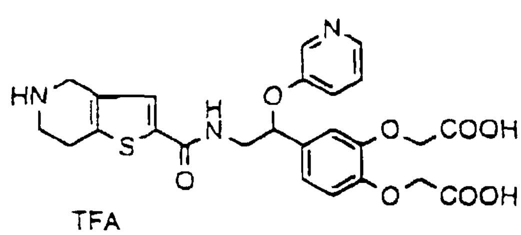

(a) Di-t-butyl [[[4-[2-[(5-t-butoxycarbonyl-4,5,6, 7-tetrahydrothieno[3,2-c]pyridin-2-yl)carbonyl]amino-(1-hydroxy)ethyl]]-o-phenylene-]dioxy] diacetate (100 mg, synthesized according to a method described in WO 9421599) is dissolved in THF (2 ml). 3-Hydroxypyridine (14.3 mg), triphenylphosphine (47 mg), and diethyl azodicarboxylate (0.028 ml) were added to the solution, and the mixture was stirred at room temperature overnight. The solvent was removed under reduced pressure, water was added to the residue, and the mixture was extracted with ethyl acetate. The extract was washed with saturated saline and dried over magnesium sulfate, and the solvent was removed under reduced pressure. The residue was purified by column chromatography on silica gel (5 g, n-hexane : ethyl acetate = 1 : 2) to give 19.8 mg (18.0%) of di-t-butyl [[4-[2-[(5-t-butoxycarbonyl-4,5,6,7-tetrahydrothieno[3,2-c]pyridin-2-yl)carbonyl]amino-1-[(pyridin-3-yl)oxy]ethyl]-o-phenylene]dioxy] diacetate.

$^1$H-NMR ($CDCl_3$) δ: 1.45 (27H, s), 1.97 (2H, brs), 2.85 (2H, brs), 3.72 (2H, brs), 4.21 (2H, dd, J = 26.0, 29.0 Hz), 4.61 (4H, s), 5.30 (1H, dd, J = 10.0, 29.0 Hz), 6.81 (1H, m), 6.91 (1H, m), 7.15 (2H, m), 7.49 (2H, m), 8.13 (1H, d, J = 8.0 Hz), 8.30 (1H, s)
ESIMS (m/z): 740 ($M^+$ + 1)

(b) The compound (19.8 mg) prepared in the above step (a) was dissolved in methylene chloride (0.1 ml), and anisole (0.01 ml) was added thereto. Trifluoroacetic acid (0.1 ml) was added to the solution under ice cooling, and the mixture was stirred for 30 min and then stirred at room temperature for additional one hr. The solvent was removed under reduced pressure, and the residue was dissolved in water. The solution was washed with isopropyl ether and then lyophilized to give 11.0 mg (65.0%) of the title compound. $^1$H-NMR ($D_2O$) δ: 3.12 (2H, brs), 3.53 (2H, brs), 3.89 (2H, brs), 4.24 (2H, brs), 4.61 (4H, m), 5.62 (1H, brs), 6.97 (2H, brs), 7.81 (1H, brs), 7.90 (1H, brs), 8.04 (1H, brs), 8.28 (1H, brs), 8.39 (1H, brs), 8.81 (1H, brs)

FDMS (m/z): 528 ($M^+$ + 1)

## Example 4

[[4-[2-[[(4,5,6,7-Tetrahydrothieno[3,2-c]pyridin-2-yl)carbonyl]-N-methylamino]-1-(pyridin-3-yl)oxyethyl]-o-phenylene]dioxy]diacetic acid trifluoroacetate

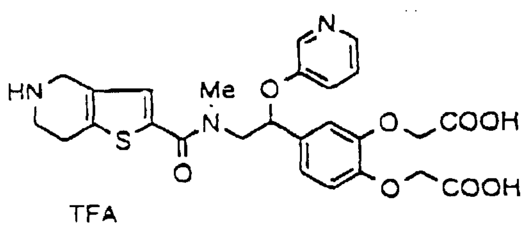

(a) Di-t-butyl [[4-[[[(5-t-butoxycarbonyl-4,5,6,7-tetrahydrothieno[3,2-c]pyridin-2-yl)carbonyl]-N-methylamino]acetyl]-o-phenylene]dioxy] diacetate (2.20 g, synthesized according to a method described in WO 9421599) was dissolved in ethanol. Sodium borohydride (49.3 mg) was added to the solution under ice cooling, and the mixture was stirred for one hr. Water was added to the reaction solution, and the mixture was extracted with ethyl acetate. The extract was washed with water and saturated saline and dried over sodium sulfate. The solvent was removed under reduced pressure, and the residue was purified by column chromatography on silica gel (65 g, n-hexane : ethyl acetate = 1 : 1) to give 1.82 g (83 %) of di-t-butyl [[4-[2-[[(5-t-butoxycarbonyl-4,5,6,7-tetrahydrothieno[3,2-c]pyridin-2-yl)carbonyl]-N-methylamino]-1-hydroxyethyl]-o-phenylene]dioxy]diacetate.

$^1$H-NMR ($CDCl_3$) δ: 1.51 (27H, s), 2.87 (2H, brs), 3.19 (3H, s), 3.62 (2H, brs), 3.78 (2H, brs), 4.50 (2H, s), 4.62 (4H, s), 5.00 (1H, brs), 6.84 (1H, d, J = 2.6 Hz), 6.98 (2H, d, J = 2.6 Hz), 7.13 (1H, s)
FDMS (m/z): 676 ($M^+$)

(b) The compound (100 mg) prepared in the above step (a) was dissolved in dehydrated THF. 3-Hydroxypyridine (14 mg), triphenylphosphine (46.6 mg), and diethyl azocarboxylate (0.027 ml) were added to the solution. The mixture was stirred at room temperature overnight. The solvent was removed under reduced pressure. Ethyl acetate

and water were added to the residue, followed by separation. The organic layer was washed with saturated saline and dried over magnesium sulfate, and the solvent was removed under reduced pressure. The residue was purified by column chromatography on silica gel (3.5 g, n-hexane : ethyl acetate = 1 : 4) to give 47.8 mg (43%) of di-t-butyl [[4-[2-[[(5-t-butoxycarbonyl-4,5,6,7-tetrahydrothieno[3,2-c]pyridin-2-yl)carbonyl]-N-methylamino]-1-(pyridin-3-yl)oxyethyl]-o-phenylene]dioxy]diacetate.

$^1$H-NMR ($CDCl_3$) δ: 1.48 (27H, s), 2.86 (2H, brs), 3.30 (3H, s), 3.71 (3H, brs), 3.96 (1H, brs), 4.48 (2H, s), 4.61 (4H, s), 5.50 (1H, brs), 6.82 (1H, d, J = 2.6 Hz), 6.98 (2H, brs), 7.08 (3H, s), 8.13 (1H, t, J = 1.3 Hz), 8.30 (1H, s)
FDMS (m/z): 753 ($M^+$)

(c) The compound (47.8 mg) prepared in the above step (b) was dissolved in methylene chloride (0.2 ml). Anisole (0.02 ml) was added to the solution. Trifluoroacetic acid (0.2 ml) was added to this solution under ice cooling, and the mixture was stirred for 30 min and then stirred at room temperature for additional one hr. The solvent was removed under reduced pressure, and the residue was dissolved in water. The solution was washed with isopropyl ether and then lyophilized to give 41.6 mg (100%) of the title compound.

$^1$H-NMR ($D_2O$) δ: 3.11 (2H, brs), 3.27 (3H, s), 3.58 (2H, brs), 4.28 (4H, s), 4.70 (2H, brs), 5.69 (1H, brs), 6.90 (4H, brs), 7.87 (1H, brs), 8.06 (1H, brs), 8.34 (2H, brs)
FDMS (m/z): 541 ($M^+$)

Example 5

[4-[2-[[(4,5,6,7-Tetrahydrothieno[3,2-c]pyridin-2-yl)carbonyl]amino]-1-(pyridin-3-yl)oxyethyl]phenyl]oxyacetic acid trifluoroacetate

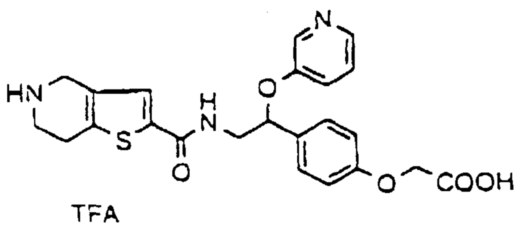

(a) t-Butyl (4-bromoacetylphenyl)oxyacetate (1.929 g) was dissolved in N,N-dimethylformamide (20 ml), sodium azide (457 mg) was added to the solution, and the mixture was stirred at room temperature for one hr. Ethyl acetate was added to the reaction solution. The mixture was washed with water and dried over magnesium sulfate, and the solvent was removed under reduced pressure. The residue was purified by column chromatography on silica gel (52 g, n-hexane : ethyl acetate = 3 : 1) to give 1.707 g (100%) of t-butyl (4-azidoacetylphenyl)oxyacetate.

$^1$H-NMR ($CDCl_3$) δ: 1.41 (9H, s), 4.50 (2H, s), 4.59 (2H, s), 6.95 (2H, d, J = 3.0 Hz), 7.89 (2H, d, J = 3.0 Hz)
EIMS (m/z): 291 ($M^+$)

(b) The compound (2.234 g) prepared in the above step (a) was dissolved in ethanol (40 ml). 10% palladium-carbon (230 mg) and concentrated hydrochloric acid (0.64 ml) were added to the solution. The mixture was stirred in a hydrogen atmosphere at atmospheric pressure for 2 hr. The reaction solution was filtered, and the filtrate was concentrated. The residue was dissolved in water, and the solution was washed with diethyl ether and then lyophilized to give 1.709 g (74.0%) of t-butyl (4-aminoacetylphenyl)oxyacetate hydrochloride.

$^1$H-NMR ($D_2O$) δ: 1.50 (9H, s), 4.66 (2H, s), 4.82 (2H, s), 7.12 (2H, d, J = 3.0 Hz), 8.03 (2H, d, J = 3.0 Hz)
ESIMS (m/z): 266 ($M^+$ + 1)

(c) 5-t-Butoxycarbonyl-4,5,6,7-tetrahydrothieno [3,2-c]pyridin-2-carboxylic acid (939 mg) was dissolved in methylene chloride (10 ml). BOP (1.47 g) was added to the solution, and the mixture was stirred at room temperature for

one hr. A solution of the compound (1 g), prepared in the above step (b), dissolved in methylene chloride (5 ml) was added thereto, and diisopropylethylamine (1.4 ml) was further added thereto. The mixture was stirred at room temperature for 4 hr. The reaction solution was concentrated, and the residue wad dissolved in ethyl acetate. The solution was washed with 0.5 N hydrochloric acid, water, and an aqueous saturated sodium bicarbonate solution in that order and then dried over magnesium sulfate. The solvent was removed under reduced pressure, and the residue was purified by column chromatography on silica gel (70 g, n-hexane : ethyl acetate = 4 : 3) to give 1.572 g (89.0%) of t-butyl [4-[[[5-(t-butoxycarbonyl)-4,5,6,7-tetrahydrathieno[3,2-c]pyridin-2- yl]carbonylamino]acetyl]phenyl]oxyacetate.

$^1$H-NMR ($CDCl_3$) δ: 1.50 (18H, s), 2.88 (2H, brs), 3.74 (2H, brs), 4.50 (2H, s), 4.61 (2H, s), 4.86 (2H, d, J = 1.4 Hz), 6.97 (2H, d, J = 2.4 Hz), 7.33 (1H, s), 7.99 (2H, d, J = 2.8 Hz)
FDMS (m/z): 530 ($M^+$)

(d) The compound (1.572 g) prepared in the above step (c) was dissolved in ethanol (16 ml). Sodium borohydride (45 mg) was added to the solution under ice cooling. The mixture was stirred for one hr. Cold water was added to the reaction solution, and the mixture was extracted with ethyl acetate. The extract was washed with water and saturated saline. The organic layer was dried over magnesium sulfate, and the solvent was removed under reduced pressure to give 1.574 g (100%) of t-butyl [4-[2-[[[5-(t-butoxycarbonyl)-4,5,6,7-tetrahydrothieno[3,2-c]pyridin-2-yl]carbonyl]amino]-1-hydroxyethyl]phenyl]oxyacetate.

$^1$H-NMR ($CDCl_3$) δ: 1.48 (18H, s), 2.85 (2H, brs), 3.42 (1H, m), 3.71 (2H, brs), 3.80 (1H, m), 4.45 (2H, s), 4.51 (2H, s), 4.86 (1H, m), 6.87 (2H, d, J = 3.0 Hz), 7.19 (1H, s), 7.30 (2H, d, J = 3.0 Hz)
FDMS (m/z): 532 ($M^+$)

(e) The compound (100 mg) prepared in the above step (d) was dissolved in dehydrated THF (2 ml). 3-Hydroxypyridine (18 mg), triphenylphosphine (59 mg), and diethyl azodicarboxylate (0.04 ml) were added to the solution, and the mixture was stirred at room temperature overnight. The solvent for the reaction was removed under reduced pressure. Water was added to the residue, and the mixture was extracted with ethyl acetate. The extract was washed with saturated saline and dried over magnesium sulfate, and the solvent was removed under reduced pressure. The residue was purified by column chromatography on silica gel (5 g, n-hexane : ethyl acetate = 1 : 4) to give 48.8 mg (43.0%) of t-butyl [4-[2-[[[5-(t-butoxycarbonyl)-4,5,6,7-tetrahydrathieno[3,2-c]pyridin-2-yl]carbonyl]amino]-1-[(pyridin-3-yl)oxy]ethyl]phenyl]oxyacetate.

$^1$H-NMR ($CDCl_3$) δ: 1.48 (18H, s), 2.82 (2H, brs), 3.62 (1H, m), 3.71 (2H, brs), 3.96 (1H, m), 4.46 (2H, s), 4.51 (2H, s), 5.37 (1H, m), 6.90 (2H, d, J = 3.0 Hz), 7.18 (2H, m), 7.45 (2H, m), 7.61 (1H, m), 8.12 (1H, s), 8.29 (1H, s)
FDMS (m/z): 609 ($M^+$)

(f) The compound (48.8 mg) prepared in the above step (e) was dissolved in methylene chloride (0.25 ml), and anisole (0.025 ml) was added to the solution. Trifluoroacetic acid (0.25 ml) was added to the solution under ice cooling, and the mixture was stirred for 1.5 hr. The solvent was removed under reduced pressure. The residue was dissolved in water, and the solution was washed with isopropyl ether and lyophilized to give 10.9 mg (24.0%) of the title compound.

$^1$H-NMR ($D_2O$) δ: 3.18 (2H, brs), 3.51 (2H, brs), 4.28 (4H, m), 4.61 (2H, m), 6.71 (1H, m), 6.92 (2H, m), 7.10 (1H, m), 7.29 (2H, m), 7.60 (1H, m), 7.99 (1H, s), 8.28 (1H, m)
FABMS (m/z): 454 ($M^+$ + 1)

Example 6

[2-(1-Imidazolylmethyl)-4-[[4-(piperidin-4-yl)-2-oxopiperazin-1-yl]acetyl]phenyl]oxyacetic acid trifluoroacetate

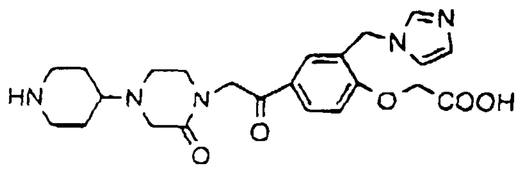

TFA

(a) 4'-Hydroxy-3'-methylacetophenone (10.5 g) was dissolved in N,N-dimethylformamide (140 ml). Potassium carbonate (11.6 g) and t-butyl bromoacetate (11.3 ml) were added to the solution, and the mixture was stirred at room temperature for 3 hr. Water was added to the reaction solution, and the mixture was extracted with ethyl acetate. The organic layer was washed with saturated saline and dried over magnesium sulfate. The solvent was removed under reduced pressure. The residue was recrystallized from hexane to give 17.2 g (93.0%) of t-butyl (4-acetyl-2-methylphenyl)oxyacetate.

$^1$H-NMR ($CDCl_3$) δ: 1.48 (9H, s), 2.32 (3H, s), 2.55 (3H, s), 4.61 (2H, s), 6.69 (1H, d, J = 8.1 Hz), 7.78 (1H, d, J = 8.1 Hz), 7.80 (1H, s).
EIMS (m/z): 264 ($M^+$)

(b) The compound (13.2 g) prepared in the above step (a) was dissolved in carbon tetrachloride (300 ml). N-bromosuccinimide (8.90 g) and 2,2'-azobisisobutyronitrile (0.30 g) were added to the solution. The mixture was heated under reflux with stirring for 2 hr. Insolubles were removed by filtration, and the solvent was then removed under reduced pressure. The residue was purified by column chromatography on silica gel (550 g, n-hexane : ethyl acetate = 5 : 1 to 3 : 1) to give 11.1 g (65.0%) of t-butyl (4-acetyl-2-bromomethylphenyl)oxyacetate.

$^1$H-NMR ($CDCl_3$) δ: 1.48 (9H, s), 2.57 (3H, s), 4.63 (2H, s), 4.69 (2H, s), 6.78 (1H, d, J = 8.6 Hz), 7.91 (1H, dd, J = 2.2, 8.6 Hz), 8.00 (1H, d, J = 2.2 Hz)
EIMS (m/z): 342, 344 (M+-1, $M^+$ + 1)

(c) The compound (10.3 g) prepared in the above step (b) was dissolved in N,N-dimethylformamide (150 ml). Potassium acetate (3.53 g) was added to the solution, and the mixture was stirred for one hr. Water was added to the reaction solution, and the mixture was extracted with ethyl acetate. The organic layer was washed with saturated saline and dried over magnesium sulfate. The solvent was removed under reduced pressure, and the residue was purified by column chromatography on silica gel (250 g, n-hexane : ethyl acetate = 4 : 1 to 3 : 1) to give 8.54 g (88.0%) of t-butyl (2-acetoxymethyl-4-acetylphenyl)oxyacetate.

$^1$H-NMR ($CDCl_3$) δ: 1.47 (9H, s), 2.14 (3H, s), 2.57 (3H, s), 4.64 (2H, s), 5.25 (2H, s), 6.78 (1H, d, J = 8.6 Hz), 7.92 (1H, dd, J = 2.2, 8.6 Hz), 7.99 (1H, d, J = 2.2 Hz).
EIMS (m/z): 322 ($M^+$)

(d) The compound (8.53 g) prepared in the above step (c) was dissolved in dichloroethane (60 ml). Triethylamine (11 ml) and trimethylsilyl trifluoromethanesulfonate (6.1 ml) were added to the solution under ice cooling. The mixture was stirred at room temperature for 30 min. The solvent was removed under reduced pressure. The residue was decanted with ether, followed under reduced pressure to remove ether. The residue was dissolved in tetrahydrofuran (60 ml). N-bromosuccinimide (5.2 g) was added to the solution under ice cooling, and the mixture was stirred for one hr. The reaction solution was poured into a cold aqueous saturated sodium bicarbonate solution under ice cooling, and the mixture was extracted wit ether. The organic layer was washed with saturated saline and dried over magnesium sulfate. The solvent was removed under reduced pressure, and the residue was purified by column chromatography on silica gel (400 g, n-hexane : ethyl acetate = 4 : 1) to give 7.68 g (72.0%) of t-butyl (2-acetoxymethyl-4-bromoacetylphenyl)oxyacetate.

$^1$H-NMR ($CDCl_3$) δ: 1.47 (9H, s), 2.15 (3H, s), 4.41 (2H, s), 4.65 (2H, s), 5.25 (2H, s), 6.80 (1H, d, J = 8.9 Hz), 7.95 (1H, dd, J = 2.2, 8.9 Hz), 8.02 (1H, d, J = 2.2 Hz).
EIMS (m/z): 400, 402 ($M^+$ - 1, $M^+$ + 1)

(e) 4-(1-t-Butoxycarbonylpiperidin-4-yl)-2-oxopiperazine (368 mg, synthesized according to a method described in WO 9602503) was dissolved in tetrahydrofuran (8 ml). A 1.0 M tetrahydrofuran solution of lithium hexamethyl disilazane (1.7 ml) was added to the solution under ice cooling, and the mixture was stirred at room temperature for 30 min. A solution of the compound (522 mg), prepared in the above step (d), in tetrahydrofuran (5 ml) was added to the reaction solution under ice cooling, and the mixture was stirred for 45 min. Acetic acid and water were added to the reaction solution, and the mixture was extracted with ethyl acetate. The organic layer was washed with an aqueous saturated sodium bicarbonate solution and saturated saline and then dried over magnesium sulfate. The solvent was removed under reduced pressure. The residue was purified by column chromatography on silica gel (30 g, n-hexane : ethyl acetate = 3 : 1 to 1 : 1) to give 337 mg (43.0%) of t-butyl [2-acetoxymethyl-4-[[4-(1-t-butoxycarbonylpiperidin-4-yl)-2-oxopiperazin-1-yl]acetyl]phenyl]oxyacetate.

$^1$H-NMR ($CDCl_3$) δ: 1.36-1.52 (20H), 1.82 (2H, brd), 2.14 (3H, s), 2.42-2.52 (1H, m), 2.75 (2H, brt), 2.86 (2H, brs), 3.37 (4H, s), 4.13 (2H, brs), 4.64 (2H, s), 4.79 (2H, s), 5.24 (2H, s), 6.79 (1H, d, J = 8.6 Hz), 7.93 (1H, dd, J = 2.2, 8.6 Hz), 7.99 (1H, d, J = 2.2 Hz)
SIMS (m/z): 603 ($M^+$)

(f) The compound (330 mg) prepared in the above step (a) was dissolved in methanol (5 ml). Magnesium (13.2 mg) was added to the solution, and the mixture was stirred at room temperature for 4 hr. Ethyl acetate and saturated saline were added to the reaction solution, and insolubles were removed by suction filtration through Celite. The organic layer was washed with saturated saline and dried over magnesium sulfate. The solvent was removed under reduced pressure. The residue was purified by column chromatography on silica gel (15 g, chloroform-chloroform : methanol = 60 : 1) to give 187 mg (61.0%) of t-butyl [4-[[4-(1-t-butoxycarbonylpiperidin-4-yl)-2-oxopiperazin-1-yl]acetyl]-2-hydroxymethylphenyl]oxyacetate.

$^1$H-NMR ($CDCl_3$) δ: 1.36-1.53 (20H), 1.82 (2H, brd), 2.44-2.52 (1H, m), 2.74 (2H, brt), 2.86 (2H, brs), 3.16 (1H, brs), 3.33-3.42 (4H, m), 4.14 (2H, brs), 4.66 (2H, s), 4.76 (2H, s), 4.78 (2H, s), 6.83 (1H, d, J = 8.6 Hz), 7.93 (1H, dd, J = 2.2, 8.6 Hz), 7.95 (1H, d, J = 2.2 Hz)
FDMS (m/z): 561 ($M^+$)

(g) The compound (79 mg) prepared in the above step (f) was dissolved in methylene chloride (2 ml). Triethylamine (40 μl) and methanesulfonyl chloride (18 μl) were added to the solution under ice cooling. 1.5 hr after the addition of triethylamine and methanesulfonyl chloride, methanesulfonyl chloride (2 μl) was added thereto, followed by stirring for additional 30 min. Ethyl acetate wag added to the reaction solution to conduct extraction. The organic layer was washed with water, 0.1 N hydrochloric acid, an aqueous saturated sodium bicarbonate solution, and saturated saline in that order and dried over sodium sulfate. The solvent was removed under reduced pressure. The residue was dissolved in methylene chloride (2 ml), and imidazole (25.2 mg) and triethylamine (35 μl) were added to the solution. The mixture was stirred at room temperature for 3 days. Ethyl acetate was added to the reaction solution, and the extracted organic layer was washed with water and saturated saline and dried over sodium sulfate. The solvent was removed under reduced pressure. The residue was purified by PTLC (Merck 5744, chloroform : methanol = 25 : 1), followed by elution with chloroform : methanol = 10 : 1 to give 32 mg (31.0%) of t-butyl [4-[[4-(1-t-butoxycarbonylpiperidin-4-yl)-2-oxopiperazin-1-yl]acetyl]-2-(1-imidazolylmethyl)phenyl]oxyacetate.

$^1$H-NMR ($CDCl_3$) δ: 1.35-1.58 (20H), 1.81 (2H, brd), 2.42-2.52 (1H, m), 2.75 (2H, brt), 2.84 (2H, brs), 3.30-3.40 (4H, m), 4.13 (2H, brs), 4.63 (2H, s), 4.71 (2H, s), 5.21 (2H, s) 6.81 (1H, d, J = 8.6 Hz), 7.03 (2H, d, J = 11.4 Hz), 7.66 (1H, s), 7.73 (1H, d, J = 2.2 Hz), 7.95 (1H, dd, J = 2.2, 8.6 Hz)
FDMS (m/z): 612 ($M^+$ + 1)

(h) The compound (45.6 mg) prepared in the above step (g) was dissolved in anisole (0.25 ml) and trifluoroacetic acid (1 ml), and the solution was stirred at room temperature for 1.5 hr. Diisopropyl ether was added to the reaction solution. The precipitated crystal was collected by filtration to give 25 mg (49.0%) of the title compound.

$^1$H-NMR ($D_2O$) δ: 1.94-2.09 (2H, m), 2.48 (2H, brd), 3.15 (2H, dt, J = 2.2, 13.4 Hz), 3.63-3.80 (7H, m), 4.11 (2H, s), 4.79 (2H, s), 5.04 (2H, s), 5.51 (2H, s), 7.07 (1H, d, J = 8.8 Hz), 7.39 (1H, t, J = 1.9 Hz), 7.51 (1H, t, J = 1.9 Hz), 8.09 (1H, dd, J = 2.3, 8.8 Hz), 8.12 (1H, d, J = 2.3 Hz), 8.86 (1H, s)

FDMS (m/z): 456 ($M^+$ + 1)

Example 7

[4-[[4-(Piperidin-4-yl)-2-oxopiperazin-1-yl]acetyl]-2-(3-pyridyloxymethyl)phenyl]oxyacetic acid trifluoroacetate

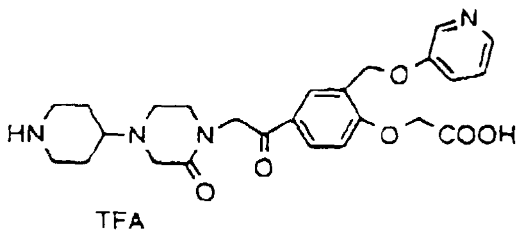

(a) The procedure of the step (g) of Example 6 was repeated, except that 3-hydroxypyridine was used instead of imidazole. Thus, 35.9 mg (34.0%) of t-butyl [4-[[4-(1-t-butoxycarbonylpiperidin-4-yl)-2-oxopiperazin-1-yl]acetyl]-2-(3-pyridyloxymethyl)phenyl]oxyacetate was prepared.

$^1$H-NMR ($CDCl_3$) δ: 1.36-1.55 (20H), 1.69-1.86 (2H, m), 2.43-2.52 (1H, m), 2.75 (2H, brt), 2.85 (2H, brt), 3.32-3.42 (4H, m), 4.13 (2H, brs), 4.64 (2H, s), 4.75 (2H, s), 5.34 (2H, s), 6.86 (1H, d, J = 8.5 Hz), 7.21 (1H, dd, J = 5.4, 9.0 Hz), 7.25-7.32 (1H, m), 7.39 (1H, d, J = 5.4 Hz), 7.59-7.63 (1H, m), 8.02 (1H, d, J = 2.5 Hz), 8.04 (1H, dd, J = 2.1, 8.5 Hz)
FDMS (m/z): 639 ($M^+$ + 1)

(b) The compound (32.0 mg) prepared in the above step (a) was treated in the same manner as in the step (h) of Example 6 to give 19.0 mg (54.0%) of the title compound.

$^1$H-NMR ($D_2O$) δ: 1.36-1.55 (20H), 1.69-1.86 (2H, m), 2.43-2.52 (1H, m), 2.75 (2H, brt), 2.85 (2H, brt), 3.32-3.42 (4H, m), 4.13 (2H, brs), 4.64 (2H, s), 4.75 (2H, s), 5.34 (2H, s), 6.86 (1H, d, J = 8.5 Hz), 7.21 (1H, dd, J = 5.4, 9.0 Hz), 7.25-7.32 (1H, m), 7.39 (1H, d, J = 5.4 Hz), 7.59-7.63 (1H, m), 8.02 (1H, d, J = 2.5 Hz), 8.04 (1H, dd, J = 2.1, 8.5 Hz)
FDMS (m/z): 639 ($M^+$ + 1)

Example 8

[2-(1-Imidazolylmethyl)-4-[[4-(piperidin-4-yl)-2,6-dioxopiperazin-1-yl]acetyl]phenyl]oxyacetic acid trifluoroacetate

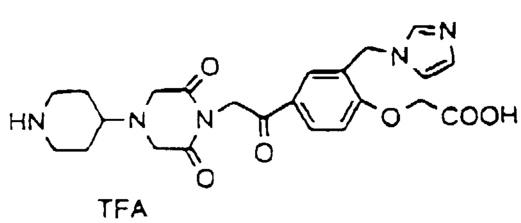

(a) Sodium hydride (0.14 g) was washed with hexane and suspended in N,N-dimethylformamide (3 ml), and a solution of 4-(1-t-butoxycarbonylpiperidin-4-yl)-2,6-dioxopiperazine (0.684 g, WO 9602503) in dimethylformamide (7 ml) was added to the suspension under ice cooling. The mixture was stirred at room temperature for 20 min. A solution of the compound (1.01 g) prepared in Example 1 (d) in N,N-dimethylformamide (5 ml) was added thereto under

ice cooling, followed by stirred for 45 min. Acetic acid and water were added to the reaction solution, and the mixture was extracted with ethyl acetate. The organic layer was washed with an aqueous saturated sodium bicarbonate solution and saturated saline and dried over magnesium sulfate, and the solvent was removed under reduced pressure. The residue was purified by column chromatography on silica gel (60 g, hexane : ethyl acetate = 1 : 1) to give 1.21 g (85.0%) of t-butyl [2-acetoxymethyl-4-[[4-(1-t-butoxycarbonylpiperidin-4-yl)-2, 6-dioxopiperazin-1-yl]acetyl]phenyl]oxyacetate.

$^1$H-NMR ($CDCl_3$) $\delta$: 1.36-1.54 (20H), 1.83 (2H, brd), 2.14 (3H, s), 2.65 (1H, tt, J = 4.0, 11.1 Hz), 2.77 (2H, brt), 3.64 (4H, s), 4.15 (2H, brs), 4.65 (2H, s), 5.16 (2H, s), 5.25 (2H, s), 6.81 (1H, d, J = 8.6 Hz), 7.93 (1H, dd, J = 2.2, 8.6 Hz), 8.00 (1H, d, J = 2.2 Hz)
FDMS (m/z): 617 ($M^+$)

(b) The compound (50 mg) prepared in the above step (a) was dissolved in methanol (1 ml). Acetyl chloride (4 µl) was added to the solution under ice cooling, and the mixture was stirred at room temperature for 27 hr. Water was added to the reaction solution, followed by extraction with ethyl acetate. The organic layer was washed with an aqueous saturated sodium bicarbonate solution and saturated saline and then dried over magnesium sulfate. The solvent was removed under reduced pressure. The residue was purified by PTLC (Merck 5744, chloroform : methanol = 20 : 1), eluting with chloroform : methanol = 10 : 1, to give 21.4 mg (46.0%) of t-butyl [4-[[4-(1-t-butoxycarbonylpiperidin-4-yl)-2-oxopiperazin-1-yl]acetyl]-2-hydroxymethylphenyl]oxyacetate.

$^1$H-NMR ($CDCl_3$) $\delta$: 1.36-1.53 (20H), 1.82 (2H, brd), 2.44-2.52 (1H, m), 2.74 (2H, brt), 2.86 (2H, brs), 3.16 (1H, brs), 3.33-3.42 (4H, m), 4.14 (2H, brs), 4.66 (2H, s), 4.76 (2H, s), 4.78 (2H, s), 6.83 (1H, d, J = 8.6 Hz), 7.93 (1H, dd, J = 2.2, 8.6 Hz), 7.95 (1H, d, J = 2.2 Hz)
FDMS (m/z): 561 ($M^+$)

(c) The compound (158 mg) prepared in the above step (b) was treated in the same manner as in Example 6 (g) to give 51.9 mg (30.0%) of t-butyl [4-[[4-(1-t-butoxycarbonylpiperidin-4-yl)-2, 6-dioxopiperazin-1-yl]acetyl]-2-(1-imidazolylmethyl)phenyl]oxyacetate.

$^1$H-NMR ($CDCl_3$) $\delta$: 1.36-1.57 (20H), 1.83 (2H, brd), 2.64 (1H, tt, J = 4.0, 11.1 Hz), 2.77 (2H, brt), 3.63 (4H, s), 4.15 (2H, brs), 4.64 (2H, s), 5.08 (2H, s), 5.22 (2H, s), 6.83 (1H, d, J = 8.7 Hz), 7.00 (1H, s), 7.06 (1H, s), 7.67 (1H, s), 7.68 (1H, d, J = 2.3 Hz), 7.94 (1H, dd, J = 2.3, 8.7 Hz)
ESIMS (m/z): 626 ($M^+$ + 1)

(d) The compound (50 mg) prepared in the above step (c) was treated in the same manner as in Example 6 (h) to give 43.4 mg (78.0%) of the title compound.

$^1$H-NMR ($D_2O$) $\delta$: 1.69-1.82 (20H), 2.21 (2H, brd), 2.97-3.13 (3H, m), 3.56 (2H, brd), 3.85 (4H, s), 4.88 (2H, s), 5.32 (2H, s), 5.51 (2H, s), 7.11 (1H, d, J = 8.8 Hz), 7.39 (1H, t, J = 1.9 Hz), 7.51 (1H, t, J = 1.9 Hz), 8.12-8.19 (2H, m), 8.86 (1H, s)
FABMS (m/z): 470 ($M^+$ + 1)

Example 9

Ethyl[4-[1-(pyridin-3-yl)oxy-2-[[(4,5,6,7-tetrahydrothieno[3,2-c]pyridin-2-yl)carbonyl]amino]ethyl]phenyl]oxyacetate hydrochloride

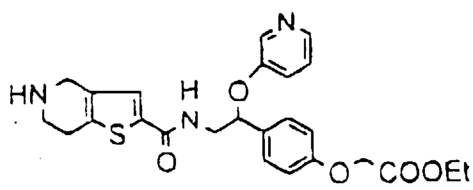

The procedure of Example 5 was repeated, except that ethyl 2-[4-(2-bromoacetyl)phenyl]oxyacetate was used instead of t-butyl 2-[4-(2-bromoacetyl)phenyl]oxyacetate. Thus, 159 mg of the title compound was prepared.

$^1$H-NMR (DMSO) δ: 1.18 (3H, t, J = 7.1 Hz), 3.04 (2H, brs), 3.37 (2H, brs), 3.55-3.63 (1H, m), 3.68-3.77 (1H, m), 4.11-4.18 (4H, m), 4.75 (2H s), 5.64-5.72 (1H, m), 6.93 (2H, d, J = 8.8 Hz), 7.40 (2H, d, J = 8.8 Hz), 7.54 (1H, s), 7.65 (1H, brs), 7.85 (1H, brd, J = 8.8 Hz), 8.90 (1H, brt, J = 5.6 Hz), 9.60 (1H, brs)
TSPMS (m/z): 482 ($M^+$ + 1)

Example 10

Sodium [4-[2-[[(4,5,6,7-tetrahydrothieno[3,2-c]pyridin-2-yl)carbonyl]amino]-1-(pyridin-3-yl)ethyl]phenyl]oxyacetic acid

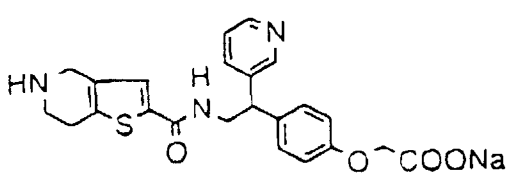

(a) 4-Bromophenol (1 g) was dissolved in methylene chloride (10 ml). Diisopropylethylamine (1.1 ml) and chloromethyl methyl ether (475 μl) were added to the solution under ice cooling, and the mixture was stirred for one hr. Methylene chloride was further added thereto, and the mixture was extracted. The organic layer was washed with saturated saline and dried over magnesium sulfate. The solvent was removed under reduced pressure, and the residue was purified by column chromatography on silica gel (50 g, n-hexane : ethyl acetate = 5 : 1) to give 774 mg (61.7%) of bromo-4-methoxymethyloxybenzene.

$^1$H-NMR ($CDCl_3$) δ: 3.43 (3H, s), 5.11 (2H, s), 6.90 (2H, d, J = 8.6 Hz), 7.35 (2H, d, J = 8.6 Hz)
EIMS (m/z): 216 ($M^+$ - 1), 218 ($M^+$ + 1)

(b) The compound (774 mg) prepared in the above step (a) was dissolved in dehydrated tetrahydrofuran (5 ml). n-Butyl lithium (1.64 M hexane solution) (2.8 ml) was added to the solution at -78°C, and the mixture was stirred for 15 min. A solution of 3-pyridylaldehyde (335 μl) in THF (3 ml) was added thereto, and the mixture was stirred for 20 min. The temperature was returned at room temperature, and stirring was continued for additional 2.5 hr. Water was added to the reaction solution, and the mixture was extracted with ethyl acetate. The organic layer was dried over magnesium sulfate, and the solvent was removed under reduced pressure. The residue was purified by column chromatography on silica gel (40 g, ethyl acetate) to give 633 mg (72.4%) of α-(4-methoxymethyloxyphenyl)-

3-pyridinemethanol.

$^1$H-NMR ($CDCl_3$) δ: 3.44 (3H, s), 5.13 (2H, s), 5.52 (2H, brs), 6.98 (2H, d, J = 8.6 Hz), 7.16-7.21 (1H, m), 7.24 (2H, d, J = 8.6 Hz), 7.68 (1H, dd, J = 1.8, 7.9 Hz), 8.27 (1H, dd, J = 1.6, 6.5 Hz), 8.41 (2H, d, J = 2.0 Hz)
EIMS (m/z): 245 ($M^+$)

(c) The compound (633 mg) prepared in the above step (b) was dissolved in chloroform (30 ml). Active manganese dioxide (1.58 g) was added thereto, followed by stirring at room temperature overnight. Manganese dioxide was removed from the reaction solution by filtration. The solvent was then removed under reduced pressure. The residue was purified by column chromatography on silica gel (30 g, ethyl acetate) to give 427 mg (68.0%) of 4-methoxymethyloxyphenyl-3-pyridyl ketone.

$^1$H-NMR ($CDCl_3$) δ: 3.38 (3H, s), 5.15 (2H, s), 7.02 (2H, d, J = 8.7 Hz), 7.33 (1H, dd, J = 4.7, 7.9 Hz), 7.70 (2H, d, J = 8.7 Hz), 7.96 (1H, d, J = 7.9 Hz), 8.67 (1H, dd, J = 1.6, 4.7 Hz), 8.85 (1H, d, J = 1.9 Hz)
EIMS (m/z): 243 ($M^+$)

(d) Metallic sodium (61 mg) was added to and completely dissolved in ethanol (5 ml). A solution of diethylcyanomethyl phosphonate (312 μl) in ethanol (2 ml) was added to the solution, and the mixture was stirred at room temperature for 15 min. Further, a solution of the compound (427 mg), prepared in the above step (c), in ethanol (2 ml) was added thereto, and the mixture was heated under reflux with stirring for 10 hr. The reaction solution was allowed to cool, and the solvent was removed under reduced pressure. The residue was extracted with ethyl acetate, and the organic layer was washed with saturated saline and dried over magnesium sulfate. The solvent was removed under reduced pressure, and the residue was purified by column chromatography on silica gel (20 g, n-hexane : ethyl acetate = 1 : 1) to give 272 mg (58.2%) of (E+Z)-3-(4-methoxymethyloxyphenyl)-3-(3-pyridyl)-2-propenenitrile.

$^1$H-NMR ($CDCl_3$) δ: 3.44 (3H, s), 5.17 (2H, s), 5.64 and 5.76 (1H each, s), 7.01 and 7.08 (2H each, d, J = 8.8 Hz), 7.20 and 7.37 (2H each, d, J = 8.8 Hz), 7.31 and 7.38 (1H each, dd, J = 4.9, 7.9 Hz), 7.56 and 7.77 (1H each, dd, J = 1.9, 7.9 Hz), 8.57 and 8.60 (1H each, d, J = 2.3 Hz), 8.64 and 8.67 (1H each, dd, J = 1.6, 4.8 Hz)
EIMS (m/z): 266 ($M^+$)

(e) The compound (395 mg) prepared in the above step (d) was dissolved in ethanol (10 ml), sodium borohydride (393 mg) was added thereto. The mixture was heated under reflux with stirring for 5 hr. The solvent for the reaction was removed under reduced pressure, and water was added thereto, followed by extraction with methylene chloride. The organic layer was washed with saturated saline and dried over magnesium sulfate, and the solvent was removed under reduced pressure. The residue was purified by column chromatography on silica gel (15 g, n-hexane : ethyl acetate = 1 : 1) to give 176 mg (44.2%) of 3-(4-methoxymethyloxyphenyl)-3-(3-pyridyl)propanenitrile.

$^1$H-NMR ($CDCl_3$) δ: 3.03 (2H, d, J = 7.6 Hz), 3.44 (3H, s), 4.37 (1H, t, J = 7.9 Hz), 5.14 (2H, s), 7.02 (2H, d, J = 8.7 Hz), 7.15 (2H, d, J = 8.7 Hz), 7.25 (1H, dd, J = 4.8, 7.9 Hz), 7.55 (1H, d, J = 7.9 Hz), 8.51 (1H, d, J = 3.8 Hz), 8.52 (1H, d, J = 4.8 Hz)
TSPMS (m/z): 269 ($M^+$ + 1)

(f) The compound (176 mg) prepared in the above step (e) was dissolved in ethylene glycol (3 ml). Potassium hydroxide (184 mg) was added to the solution. The mixture was heated under reflux with stirring for 5 hr. Water was added to the reaction solution, and the mixture was washed with ethyl acetate. The aqueous layer was neutralized with 1 N hydrochloric acid and extracted with ethyl acetate. The organic layer was washed with saturated saline and dried over magnesium sulfate. The solvent was removed under reduced pressure to give 86 mg (45.6%) of 3-(4-methoxymethyloxyphenyl)-3-(3-pyridyl)propanoic acid.

$^1$H-NMR ($CDCl_3$) δ: 2.98 (2H, d, J = 7.7 Hz), 3.36 (3H, s), 4.50 (1H, t, J = 7.7 Hz), 5.05 (2H, s), 6.89 (2H, d, J = 8.7 Hz), 7.06 (2H, d, J = 8.7 Hz), 7.17-7.20 (1H, m), 7.55 (1H, d, J = 8.0 Hz), 8.29 (1H, d, J = 3.9 Hz), 8.46 (1H, d, J = 1.2 Hz)
TPSMS (m/z): 288 ($M^+$ + 1)

(g) The compound (705 mg) prepared in the above step (f) was dissolved in dehydrated tetrahydrofuran (10 ml). Triethylamine (476 μl) and pivaloyl chloride (389 μl) were added thereto. The mixture was stirred under ice cooling for 30 min. Aqueous ammonia (448 μl) was added thereto, followed by stirring for additional one hr. Water was

added to the reaction solution. The mixture was extracted with ethyl acetate, and the extract was washed with an aqueous saturated sodium bicarbonate solution and dried over magnesium sulfate. The solvent was removed under reduced pressure to give 409 mg (58.2%) of 3-(4-methoxymethyloxyphenyl)-3-(3-pyridyl)propanamide.

$^1$H-NMR ($CDCl_3$) δ: 2.93 (2H, d, J = 7.7 Hz), 3.44 (3H, s), 4.57 (1H, t, J = 7.7 Hz), 5.12 (2H, s), 6.39 (1H, brs), 6.48 (1H, brs), 6.98 (2H, d, J = 8.7 Hz), 7.14 (1H, m), 7.22 (2H, d, J = 8.7 Hz), 7.55 (1H, d, J = 8.0 Hz), 8.41 (1H, brs), 8.52 (1H, brs)
ESIMS (m/z): 287 ($M^+$ + 1)

(h) A suspension of the compound (409 mg), prepared in the above step (g), in a 1 N aqueous sodium hydroxide solution (1.2 ml) was added to a mixed solution composed of an aqueous sodium hypochlorite solution (1 ml) and a 1 N aqueous sodium hydroxide solution (2.4 ml), and the mixture was stirred at room temperature for one hr. Sodium hypochloride (1 ml) was then added thereto, followed by stirring at room temperature for additional one hr. Thereafter, the temperature was gradually raised, and heating under reflux was carried out with stirring for 15 min. The reaction solution was allowed to cool, and water was added thereto under ice cooling. The pH was adjusted to 3-4 by the addition of 1 N hydrochloric acid. The neutralized solution was washed with ethyl acetate, and water was removed under reduced pressure. In order to remove sodium chloride, extraction with ethanol was carried out. The solvent was removed under reduced pressure to give 162 mg (43.9%) of 2-(4-methoxymethyloxyphenyl)-2-(3-pyridyl) -1-ethylamine.

$^1$H-NMR ($CD_3OD$) δ: 3.47 (3H, s), 3.79 (2H, dd, J = 8.0, 12.5 Hz), 4.28 (1H, t, J = 8.0 Hz), 5.19 (2H, s), 7.05 (2H, d, J = 8.7 Hz), 7.27 (2H, d, J = 8.7 Hz), 7.40-7.45 (1H, m), 7.82 (1H, d, J = 8.0 Hz), 8.42 (1H, brs), 8.50 (1H, brs)
ESIMS (m/z): 259 ($M^+$ + 1)

(i) 5-(9-Fluorenylmethyloxycarbonyl)-4,5,6,7-tetrahydrothieno[3,2-c]pyridine-2-carboxylic acid (223 mg) was dissolved in methylene chloride (10 ml). BOP(267 mg) was added to the solution, and the mixture was stirred at room temperature for one hr. A solution of the compound (162 mg), prepared in the above step (h), in a mixed solution composed of methylene chloride (5 ml) and N,N-dimethylformamide (1 ml) was added to the stirred solution. Diisopropylethylamine (335 μl) was added thereto, and the mixture was stirred at room temperature overnight. The reaction solution was concentrated, and the concentrate was dissolved in ethyl acetate. The solution was then washed with 1 N hydrochloric acid, water, and an aqueous saturated sodium bicarbonate solution and dried over magnesium sulfate. The solvent was removed under reduced pressure. The residue was purified twice by column chromatography on silica gel (30 g, ethyl acetate-10 g, n-hexane : ethyl acetate = 1 : 4) to give 45 mg (12.7%) of N-[2-(4-methoxymethyloxyphenyl)-2-(pyridin-3-yl)ethyl]-[5-(9-fluorenylmethyloxycarbonyl)-4,5,6,7- tetrahydrothieno[3,2-c]pyridin-2-yl]formamide.

$^1$H-NMR ($CDCl_3$) δ: 2.75 (2H, brs), 3.46 (3H, s), 3.70 (2H, brs), 4.23 (2H, brs), 4.34-4.60 (5H, m), 5.15 (2H, s), 7.02 (2H, brs), 7.17 (1H, s), 7.26-7.39 (8H, m), 7.56 (2H, brs), 7.67 (1H, brs), 7.76 (1H, brs), 8.47 (1H, brs), 8.53 (1H, brs)
FABMS (m/z): 646 ($M^+$ + 1)

(j) The compound (45 mg) prepared in the above step (i) was dissolved in tetrahydrofuran (1 ml). Concentrated hydrochloric acid (0.1 ml) was added thereto, and the mixture was stirred at 65°C for 2.5 hr. The solvent for the reaction was removed under reduced pressure. Water was added to the residue, and the mixture was neutralized with a 1 N aqueous sodium hydroxide solution, followed by extraction with ethyl acetate. The organic layer was washed with saturated saline and dried over magnesium sulfate. The solvent was removed under reduced pressure to give 42 mg (100%) of N-[2-(4-hydroxyphenyl)-2-(pyridin-3-yl)ethyl]-[5-(9-fluorenylmethyloxycarbonyl)-4,5,6,7-tetrahydrothieno[3,2-c]pyridin-2-yl]formamide.

FABMS (m/z): 602 ($M^+$ + 1)

(k) The compound (42 mg) prepared in the above step (j) was dissolved in N,N-dimethylformamide (1 ml). Anhydrous potassium carbonate (9.7 mg) and bromoethyl acetate (7.8 μl) were added to the solution, and the mixture was stirred at room temperature overnight. Water was added to the reaction solution, followed by extraction with ethyl acetate. The organic layer was washed with saturated saline and dried over magnesium sulfate, and the solvent was removed under reduced pressure to give 40 mg (83.3%) of ethyl [4-[2-[[5-(9-fluorenylmethyloxycarbonyl)-4,5,6,7-tetrahydrothieno[3,2-c]pyridin-2-yl]carbonyl]amino]-1-[(pyridin-3-yl)ethyl]phenyl[oxyacetate.

ESIMS (m/z): 688 ($M^+$ + 1)

(l) The compound (40 mg) prepared in the above step (k) was dissolved in ethanol (2 ml). 1 N sodium hydroxide (0.17 ml) was added to the solution, and the mixture was stirred at room temperature for 3.5 hr. The solvent for the reaction was removed under reduced pressure. The residue was dissolved in water. The solution was washed with isopropyl ether. Water was removed under reduced pressure. The residue was purified by HP-20 resin (5 ml) and lyophilized to give 16 mg (59.9%) of the title compound.

$^1$H-NMR ($D_2O$) δ: 3.04 (2H, m), 3.32 (2H, m), 4.07 (4H, m), 4.80 (1H, t, J = 7.7 Hz), 5.23 (2H, s), 6.92 (2H, d, J = 8.7 Hz), 7.25 (1H, s), 7.30 (2H, d, J = 8.7 Hz), 7.88 (1H, brs), 8.05 (1H, brs), 8.46 (1H, brs), 8.55 (1H, brs)
EIMS (m/z): 459 ($M^+$)

Example 11

[4-[2-[[(4,5,6,7-Tetrahydrothieno[3,2-c]pyridin-2-yl)carbonyl]-N-methylamino]-1-(pyridin-3-yl)oxyethyl]phenyl]oxyacetic acid trifluoroacetate

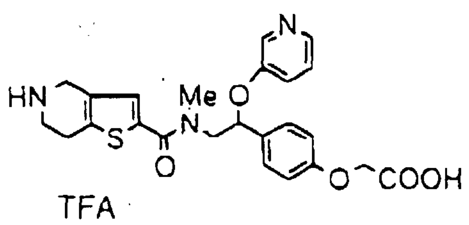

(a) N-Methylbenzylamine (78 μl) was dissolved in acetone (5 ml). Anhydrous potassium carbonate (84 mg) and t-butyl 2-[4-(2-bromoacetyl)phenyl]oxyacetate (200 mg) were added to the solution. The mixture was stirred at room temperature overnight. Water was added to the reaction solution. The mixture was extracted with ethyl acetate. The organic layer was washed with saturated saline and dried over magnesium sulfate. The solvent was removed under reduced pressure to give 220 mg (98.0%) of t-butyl 2-[4-(benzylmethylamino)acetylphenyl]oxyacetate.

$^1$N-NMR ($CDCl_3$) δ: 1.48 (9H, s), 2.35 (3H, s), 3.66 (2H, s), 3.73 (2H, s), 4.59 (2H, s), 6.89 (2H, d, J = 9.0 Hz), 7.23-7.36 (5H, m), 7.96 (2H, d, J = 9.0 Hz)
FABMS (m/z): 370 ($M^+$ + 1)

(b) The compound (220 mg) prepared in the above step (a) was dissolved in chloroform (3 ml). Benzyloxycarbonyl chloride (212 μl) and potassium hydrogencarbonate (149 mg) were added to the solution, and the mixture was stirred at room temperature overnight. Water was added to the reaction solution, and the mixture was extracted with methylene chloride. The organic layer was washed with saturated saline and dried over magnesium sulfate. The solvent was removed under reduced pressure. The residue was purified by column chromatography on silica gel (15 g, n-hexane : ethyl acetate = 3: 1) to give 175 mg (71.1%) of t-butyl 2-[4-(2-phenylmethoxy-N-methylcarbonylamino) acetylphenyl] oxyacetate.

$^1$H-NMR ($CDCl_3$) δ: 1.47 (9H, s), 3.00 (3H, s), 4.56 (4H, s), 5.26 (2H, s), 6.91 (2H, d, J = 8.8 Hz), 7.28-7.39 (5H, m), 7.92 (2H, d, J = 8.8 Hz)
TSPMS (m/z): 414 ($M^+$ + 1)

(c) The compound (175 mg) prepared in the above step (b) was dissolved in 1,4-dioxane (5 ml). 10% palladium-carbon (20 mg) was added to the solution, and the mixture was subjected to catalytic reduction with hydrogen at room temperature under atmospheric pressure overnight. The palladium-carbon was removed by filtration. The solvent for the reaction was removed under reduced pressure to give 113 mg (94.9%) of t-butyl 2-[4-[1-hydroxy-2-(methylamino)ethyl]phenyl]oxyacetate.

$^1$H-NMR ($CD_3OD$) δ: 1.48 (9H, s), 2.43 (3H, s), 3.65 (2H, s), 4.55 (2H, s), 4.73 (1H, m), 6.89 (2H, d, J = 9.0

Hz), 7.30 (2H, d, J = 9.0 Hz)
FABMS (m/z): 282 ($M^+$ + 1)

(d) 5-t-Butoxycarbonyl-4,5,6,7-tetrahydrothieno[3,2-c] pyridine-2-carboxylic acid (115 mg) was dissolved in methylene chloride (5 ml). BOP (197 mg) was added to the solution, and the mixture was stirred at room temperature for one hr. A solution of the compound (113 mg), prepared in the above step (c), in methylene chloride (2 ml) was added to the stirred solution. Diisopropylethylamine (155 μl) was added thereto, followed by stirring at room temperature overnight. Water was added to the reaction solution, and the mixture was extracted with methylene chloride. The organic layer was washed with saturated saline and dried over magnesium sulfate. The solvent was removed under reduced pressure. The residue was purified by PTLC (Merck 5744, n-hexane : ethyl acetate = 1 : 1) and elution with ethyl acetate to give 90 mg (40.7%) of t-butyl [4-[2-[[[5-(t-butoxycarbonyl)-4,5,6,7-tetrahydrothieno[3,2-c]pyridin-2-yl]carbonyl]-N-methylamino]-1-hydroxyethyl]phenyl] oxyacetate.

$^1$H-NMR ($CDCl_3$) δ: 1.49 (18H, s), 2.86 (2H, brs), 3.14 (3H, s), 3.41 (2H, s), 3.72 (2H, d, J = 5.8 Hz), 4.47 (2H, s), 4.52 (2H, s), 4.98 (1H, brs), 6.89 (2H, d, J = 8.1 Hz), 7.12 (1H, s), 7.32 (2H, d, J = 8.1 Hz)
TSPMS (m/z): 547 ($M^+$ + 1)

(e) The compound (90 mg) prepared in the above step (d) was dissolved in dehydrated tetrahydrofuran (2.5 ml). Triphenylphosphine(67 mg), diethyl azodicarboxylate (38 μl), and 3-hydroxypyridine (16 mg) were added to the solution, and the mixture was stirred at room temperature overnight. The solvent was removed under reduced pressure, water was added to the residue, followed by extraction with ethyl acetate. The organic layer was washed with saturated saline and dried over magnesium sulfate. The solvent was removed under reduced pressure. The residue was purified by PTLC (Merck 5744, ethyl acetate) and elution with ethyl acetate to give 34 mg (33.1%) of t-butyl [4-[2-[[[5-(t-butoxycarbonyl)-4,5,6,7-tetrahydrothieno[3,2-c]pyridin-2-yl]carbonyl]-N-methylamino]-1-[(pyridin-3-yl)oxy]ethyl]phenyl]oxyacetate.

$^1$H-NMR ($CDCl_3$) δ: 1.47 (9H, s), 1.49 (1H, s), 2.84 (2H, brs), 3.28 (3H, s), 3.72 (2H, brs), 3.72 (1H, brs), 3.95 (1H, brs), 4.46 (2H, s), 4.50 (2H, s), 5.55 (1H, brs), 6.88 (2H, d, J = 8.8 Hz), 7.08 (2H, d, J = 8.8 Hz), 7.10 (1H, s), 7.34 (2H, d, J = 8.0 Hz), 8.14 (1H, m), 8.28 (1H, s)
TSPMS (m/z): 624 ($M^+$ + 1)

(f) The compound (101 mg) prepared in the above step (e) was dissolved in methylene chloride (1 ml). Anisole (0.1 ml) was added to the solution. Trifluoroacetic acid (1 ml) was added thereto under ice cooling, and the mixture was stirred for 2.5 hr. The solvent for the reaction was removed under reduced pressure. The residue was dissolved in water, and the solution was washed with isopropyl ether and lyophilized to give 104 mg (92.3%) of the title compound. $^1$H-NMR ($D_2O$) δ: 3.00 (2H, m), 3.08 (3H, s), 3.39 (2H, m), 4.12 (2H, m), 4.56 (2H, s), 4.65 (2H, s), 5.59 (1H, m), 6.71 (1H, s), 6.82 (2H, d, J = 8.8 Hz), 7.00 (1H, s), 7.28 (2H, d, J = 8.8 Hz), 7.71 (1H, dd, J = 5.6, 8.8 Hz), 7.85 (1H, m), 8.12 (1H, dd, J = 5.6, 10.8 Hz)

TSPMS (m/z): 468 ($M^+$ + 1)

Example 12

[4-[1-(Pyridyl-3-ylmethyl)-2-[[(4,5,6,7-tetrahydrothieno[3,2-c]pyridin-2-yl)carbonyl]amino]ethyl]phenyl]oxyacetic acid trifluoroacetate

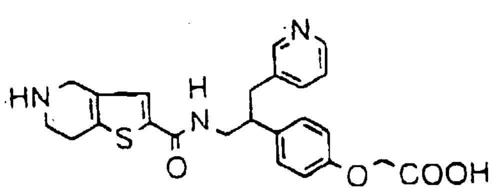

TFA

(a) 5-t-Butoxycarbonyl-4,5,6,7-tetrahydrothieno [3,2-c]pyridine-2-carboxylic acid (1.01 g) was suspended in methylene chloride (1 ml). Anisole (0.5 ml) and trifluoroacetic acid were added to the suspension. The mixture was stirred at room temperature for one hr. Diisopropyl ether was added to the reaction solution. The precipitated crystal was collected by filtration to give 1.10 g (96.0%) of 4,5,6,7-tetrahydrothieno[3,2-c]pyridine-2-carbonylic acid trifluoroacetate.

$^1$H-NMR(DMSO-$d_6$) δ: 3.16(2H, t, J = 6.0 Hz), 3.88 (2H, t, J = 6.0 Hz), 4.43(2H, s), 7.62(1H, s)

(b) The compound (1.07 g) prepared in the above step (a) was suspended in methylene chloride (22 ml). Diisopropylethylamine (3 ml) and 9-fluorenylmethylchloroformate (1.13 g) were added to the suspension under ice cooling, and the mixture was stirred for 2.5 hr. The reaction solution was poured in an aqueous ammonium chloride solution. 0.1 N Hydrochloric acid was added thereto, followed by extraction with ethyl acetate. The organic layer was dried over sodium sulfate. The solvent was removed under reduced pressure. The residue was purified by column chromatography on silica gel (100 g, methylene chloride : methanol = 20 : 1 to 10 : 1) to give 445 mg (31.0%) of 5-(9-fluorenylmethyloxycarbonyl)-4,5,6,7-tetrahydrothieno [3,2-c]pyridine-2-carboxylic acid.

$^1$H-NMR ($CDCl_3$) δ: 2.80 (2H, s) 3.72 (2H, brs), 4.26 (1H, s), 4.38 (1H, s), 4.54 (3H, s), 7.23-7.45 (5H, m), 7.56 (2H, s), 7.72 (2H, brd, J = 27.6 Hz)
EIMS (m/z): 405 ($M^+$)

(c) 4-Hydroxybenzyl cyanide (4.02 g) was dissolved in methylene chloride (40 ml). Diisopropylethylamine (8.1 ml) and chloromethyl methyl ether (2.4 ml) were added to the solution under ice cooling. The mixture was stirred for 24 hr while raising the temperature to room temperature. Water was added to the reaction solution under ice cooling, followed by extraction with methylene chloride. The organic layer was washed with an 1 N aqueous NaOH solution, water, and saturated saline in that order and dried over magnesium sulfate. The solvent was removed under reduced pressure. The residue was purified by flash column chromatography on silica gel (130 g, hexane : ethyl acetate = 3 : 1) to give 4.27 g (80.3%) of 2-[4-(methoxymethoxy)phenyl]ethanenitrile.

$^1$H-NMR ($CDCl_3$) δ: 3.48 (3H, s), 3.70 (2H, s), 5.18 (2H, s), 7.04 (2H, d, J = 8.7 Hz), 7.25 (2H, d, J = 8.7 Hz).
TSPMS (m/z): 209 ($M^+$ + 1)

(d) The compound (4.26 g) prepared in the above step (c) was dissolved in ethanol (50 ml). A solution of sodium ethoxide (165 mg) in ethanol (10 ml) was added to the solution, and the mixture was stirred at room temperature for 4 hr. About 30 ml of ethanol was removed under reduced pressure. The precipitated crystal was collected by filtration. The filtrate was concentrated. Water was added to the concentrate, followed by extraction with methylene chloride. The organic layer was washed with saturated saline and dried over sodium sulfate. The solvent was removed under reduced pressure. The resultant crystal was recrystallized from ethanol : hexane to give 5.44 g (87.1%) of 2-[4-(methoxymethoxy)phenyl]-2-(pyridin-3-ylmethylene)ethanenitrile.

$^1$H-NMR ($CDCl_3$) δ: 3.50 (3H, s), 5.23 (2H, s), 7.11 (2H, d, J = 9.0 Hz), 7.40-7.44 (2H, m), 7.63 (2H, d, J = 9.0 Hz), 8.44-8.48 (1H, m), 8.63 (1H, dd, J = 1.7, 4.9), 8.82 (1H, d, J = 1.7)
TSPMS (m/z): 267 ($M^+$ + 1)

(e) The compound (1.06 g) prepared in the above step (d) and cobalt(II) chloride hexahydrate (1.9 g) were dissolved in methanol (40 ml). Sodium borohydride (1.51 g) was added to the solution under ice cooling. The mixture was stirred at room temperature for 1.5 hr. Further, sodium borohydride (760 mg) was added thereto under ice cooling, and the mixture was stirred at room temperature for one hr. Water was added to this solution, and methanol was removed under reduced pressure. The residue was saturated with salt, followed by extraction with ether. The organic layer was dried over sodium sulfate. The solvent was removed under reduced pressure to give 860 mg (78.9%) of 3-[4-(methoxymethoxy)phenyl]-3-(pyridin-3-ylmethylene)ethylamine.

FABMS (m/z): 273 ($M^+$ + 1)

(f) The compound (1.13 g) prepared in the above step (b) was dissolved in dimethylformamide (20 ml). BOP (1.47 g) and diisopropylethylamine (1.2 ml) were added to the solution under ice cooling, and the mixture was stirred for 0.5 hr. A solution of the compound (850 mg), prepared in the above step (e), dissolved in dimethylformamide (8 ml) was added thereto at the same temperature,and the mixture was stirred for 2.5 hr. Water was added to the reaction solution under ice cooling, followed by extraction with ethyl acetate. The organic layer was washed with an aqueous

saturated sodium bicarbonate solution and saturated saline and dried over magnesium sulfate. The solvent was removed under reduced pressure. The residue was purified by flash column chromatography on silica gel (100 g, methylene chloride : methanol = 30 : 1) to give 1.10 g (53.4%) of N-[[5-(9-fluorenylmethyloxycarbonyl)-4,5,6,7-tetrahydrothieno[3,2-c]pyridin-2-yl]carbonyl]-3-[4-(methoxymethoxy)phenyl]-3-(pyridin-3-ylmethylene)ethylamine.

$^1$H-NMR ($CDCl_3$) δ: 2.75 (2H, brs), 2.86-2.96 (1H, m), 2.98-3.07 (1H, m), 3.47 (3H, s, coincident with brs, 1H), 3.71 (2H, brs), 3.93 (1H, brs), 4.25 (1H, brs), 4.35 (1H, brs), 4.49 (3H, brs), 5.15 (2H, s), 5.70 (1H, brt, J = 14.3 Hz), 6.98 (2H, d, J = 8.5 Hz, coincident with s, 1H), 7.06 (2H, brs), 7.13 (1H, dd, J = 4.9, 7.8 Hz), 7.20-7.43 (5H, m), 7.55 (2H, brs), 7.66 (1H, brs), 7.76 (1H, brs), 8.29 (1H, brs), 8.39 (1H, brd, J = 4.4 Hz)
FABMS (m/z): 660 ($M^+$ + 1)

(g) The compound (1.32 g) prepared in the above step (f) was dissolved in tetrahydrofuran (20 ml). Concentrated hydrochloric acid (0.5 ml) was added to the solution. The mixture was heated under reflux with stirring for 2 hr. The reaction solution was concentrated, and an aqueous saturated sodium bicarbonate solution was added to the concentrate, followed by extraction with methylene chloride. The organic layer was dried over sodium sulfate. The solvent was removed under reduced pressure to give 1.29 g (100%) of N-[[5-(9-fluorenylmethyloxycarbonyl)-4,5,6,7-tetrahydrothieno[3,2-c]pyridin-2-yl]carbonyl]-3-(4-hydroxyphenyl)-3-(pyridin-3-ylmethylene)ethylamine.

ESIMS(m/z): 616($M^+$ + 1)

(h) The compound (40 mg) prepared in the above step (g) was dissolved in dimethylformamide (0.7 ml). Potassium carbonate (28 mg) and di-t-butyl dicarbonate (32 μl) were added to the solution, and the mixture was stirred at room temperature for 3 hr. Water was added to the reaction solution, followed by extraction with ethyl acetate. The organic layer was washed with saturated saline and dried over sodium sulfate. The solvent was removed under reduced pressure. The residue was purified by PTLC (Merck 5744, methylene chloride : methanol = 10 : 1) and elution with methylene chloride : methanol = 10 : 1 to give 22.3 mg (57.8%) of N-[[5-(t-butyloxycarbonyl)-4,5,6,7-tetrahydrothieno [3,2-c]pyridin-2-yl]carbonyl]-3-(4-t-butyloxycarbonyloxyphenyl)-3-(pyridin-3-ylmethylene)ethylamine.

$^1$H-NMR ($CDCl_3$) δ: 1.47 (9H, s), 1.56 (9H, s), 2.82 (2H.brs), 2.87-2.94 (1H, m), 3.00-3.08 (1H, m), 3.19-3.28 (1H, m), 3.38-3.47 (1H, m), 3.69 (2H, brs), 3.89-3.97 (1H, m), 4.42 (2H, s, ), 5.71 (1H, brs), 6.99 (1H, s), 7.10-7.17 (5H, m), 7.35 (1H, d, J = 7.8 Hz), 8.32 (1H, brs), 8.39 (1H, brs)
TSPMS (m/z): 594 ($M^+$ + 1)

(i) The compound (20 mg) prepared in the above step (h) was dissolved in methanol (0.5 ml). A 1 N aqueous sodium hydroxide solution (40 μl) was added to the solution, and the mixture was stirred at room temperature for 1.5 hr. Further, a 1 N aqueous sodium hydroxide solution (27 μl) was added thereto, and the mixture was stirred at room temperature for 20 hr. The reaction solution was concentrated. Water was added to the residue. The mixture was acidified by the addition of 1 N Hydrochloric acid, and an aqueous saturated sodium bicarbonate solution was added thereto. The mixture was extracted with methylene chloride. The organic layer was dried over sodium sulfate. The solvent was removed under reduced pressure to give 16.5 mg (99.2%) of N-[[5-(t-butyloxycarbonyl)-4,5,6,7-tetrahydrothieno[3,2-c]pyridin-2-yl]carbonyl]-3-(4-hydroxyphenyl)-3-(pyridin-3-ylmethylene)ethylamine.

$^1$H-NMR ($CDCl_3$) δ: 1.47 (9H, s), 2.78-2.90 (3H, m), 3.00-3.14 (2H, m), 3.37-3.48 (1H, m), 3.60-3.75 (2H, m), 3.90-3.98 (1H, m), 4.38 (2H, s), 6.72 (2H, d, J = 8.5 Hz), 6.90 (2H, d, J = 8.5 Hz), 6.97 (1H, brs), 7.16 (1H, brt, J = 6.2 Hz), 7.42 (1H, d, J = 7.8 Hz), 8.05 (1H, brs), 8.32 (1H, brs)
TSPMS (m/z): 494 ($M^+$ + 1)

(j) The compound (70 mg) prepared in the above step (i) was dissolved in acetone (1.4 ml). Potassium carbonate (29 mg) and t-butyl bromoacetate (25 μl) were added to the solution, and the mixture was stirred at room temperature for 3 days. Water was added to the reaction solution. Acetone was removed under reduced pressure, and the residue was extracted with methylene chloride. The organic layer was washed with saturated saline and dried over sodium sulfate. The solvent was removed under reduced pressure. The residue was purified by PTLC (Merck 5744, methylene chloride : methanol = 20 : 1) and elution with methylene chloride : methanol = 10 : 1 to give 37 mg (42.9%) of t-butyl [4-[2-[[[5-(t-butoxycarbonyl)-4,5,6,7-tetrahydrothieno[3,2-c]pyridin-2-yl]carbonyl]amino]-1-(pyridin-3-ylmethylene)ethyl]phenyl]oxyacetate.

$^1$H-NMR ($CDCl_3$) δ: 1.47 (9H, s), 1.48 (9H, s), 2.78-2.91 (3H, m), 3.02 (1H, dd, J = 6.1, 13.9 Hz), 3.09-3.19 (1H, m), 3.37-3.46 (1H, m), 3.69 (2H, brs), 3.88-3.97 (1H, m), 4.42 (2H, s), 4.49 (2H, s), 5.68 (1H, brs), 6.83 (2H, d,

J = 8.5 Hz), 7.00 (1H, s), 7.05 (2H, d, J = 8.5 Hz), 7.10 (1H, dd, J = 4.9, 7.6 Hz), 7.30-7.35 (1H, m), 8.29 (1H, brs), 8.37 (1H, brd, J = 3.7 Hz)
TSPMS (m/z): 608 ($M^+$ + 1)

(k) The compound (37 mg) prepared in the above step (j) was dissolved in methylene chloride (1 ml). Anisole (40 μl) and trifluoroacetic acid (140 μl) were added to the solution under ice cooling. After 2.5 hr, trifluoroacetic acid (90 μl) was added thereto, and, after one hr, trifluoroacetic acid (270 μl) was added thereto. The mixture was stirred for 2.5 hr. Water was added to the concentrated reaction solution, and the mixture was washed with diisopropyl ether and ether. The aqueous layer was lyophilized to give 35 mg (84.6%) of the title compound.

$^1$H-NMR (DMSO) δ: 2.87-2.95 (1H, m), 2.98-3.13 (3H, m), 3.18-3.27 (1H, m), 3.34-3.50 (4H, m), 4.17 (2H, s), 4.58 (2H, s), 6.77 (2H, d, J = 8.8 Hz), 7.09 (2H, d, J = 8.8 Hz), 7.46 (1H, brs), 7.73 (1H, brd, J = 7.8 Hz), 8.50 (1H, brt, J = 5.7 Hz), 9.06 (1H, brs)
TSPMS (m/z): 452 ($M^+$ + 1)

Example 13

[4-[1-(Imidazol-1-yl)-2-[[(4,5,6,7-tetrahydrothieno[3,2-c]pyridin-2-yl)carbonyl]amino]ethyl]phenyl]oxyacetic acid trifluoroacetate

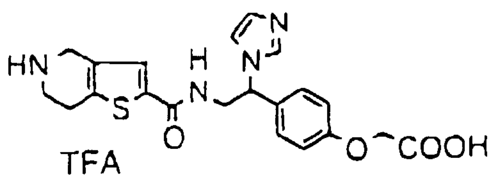

TFA

(a) 4-Hydroxyacetophenone (16.3 g) was dissolved in acetone (300 ml). Potassium carbonate (19.9 g) and methyl bromoacetate (11.6 ml) were added to the solution. The mixture was stirred at room temperature for 1.5 hr and further heated under reflux for one hr with stirring. Insolubles were removed by suction filtration, and the solvent was removed under reduced pressure. Water was added to the residue, followed by extraction with ethyl acetate. The organic layer was washed with saturated saline and dried over magnesium sulfate. The solvent was removed under reduced pressure. The residue was recrystallized from ether-hexane to give 23.1 g (93.0%) of methyl (4-acetylphenyl)oxyacetate.

$^1$H-NMR ($CDCl_3$) δ: 2.56 (3H, s), 3.82 (3H, s), 4.71 (2H, s), 6.94 (2H, d, J = 8.8 Hz), 7.94 (2H, d, J = 8.8 Hz)
TSPMS (m/z): 209 ($M^+$ + 1)

(b) The compound (10.0 g) prepared in the above step (a) was dissolved in tetrahydrofuran (120 ml). Triethylamine (13.4 ml) and trimethylsilyl trifluoromethnnesulfonate (9.6 ml) were added to the solution under ice cooling. The mixture was stirred under ice cooling for 30 min. The solvent was removed under reduced pressure. The residue was decanted with ether, and ether was removed under reduced pressure. The residue was dissolved in tetrahydrofuran (12.0 ml). N-Chlorosuccinimide (7.07 g) was added to the solution under ice cooling, and the mixture was stirred for 2 hr. Water was added to the reaction solution under ice cooling. Tetrahydrofuran was removed under reduced pressure, followed by extraction with ethyl acetate. The organic layer was washed with 10% sodium thiosulfate, an aqueous saturated sodium bicarbonate solution, and saturated saline in that order and dried over magnesium sulfate. The solvent was removed under reduced pressure. The residue was purified by flash column chromatography on silica gel (350 g, methylene chloride) to give 11.2 g (97.0%) of methyl (4-chloroacetylphenyl)oxyacetate.

$^1$H-NMR ($CDCl_3$) δ: 3.82 (3H, s), 4.64 (2H, s), 4.72 (2H, s), 6.97 (2H, d, J = 9.0 Hz), 7.96 (2H, d, J = 9.0 Hz)
TSPMS (m/z): 243, 245 ($M^+$ + 1, $M^+$ + 3)

(c) The compound (8.98 g) prepared in the above step (b) was suspended in methanol (100 ml). Sodium borohydride (400 mg) was added to the suspension under ice cooling. The mixture was stirred under ice cooling for one hr. A 10% aqueous ammonium chloride solution was added to the reaction solution under ice cooling, followed by extraction with ethyl acetate. The organic layer was washed with saturated saline and dried over magnesium sulfate. The solvent was removed under reduced pressure to give 11.2 g (97.0%) of methyl [4-(2-chloro-1-hydroxyethyl)phenyl]oxyacetate.

$^1$H-NMR ($CDCl_3$) δ: 2.60 (1H, d, J = 2.9 Hz), 3.62 (1H, dd, J = 8.8, 11.2 Hz), 3.71 (1H, dd, J = 3.7, 11.2 Hz), 3.81 (3H, s), 4.64 (2H, s), 4.83-4.88 (1H, m), 6.93 (2H, d, J = 8.8 Hz), 7.32 (2H, d, J = 8.8 Hz)
FABMS (m/z): 244, 246 ($M^+$ + 1, M++3)

(d) The compound (5.00 g) prepared in the above step (c) was dissolved in dimethylformamide (25 ml). Sodium azide (3.32 g) was added to the solution. The mixture was stirred at 90°C for 5.5 hr. Further, sodium azide (663 mg) was added thereto, and the mixture was stirred at 90°C for 1.5 hr. Water was added to the reaction solution under ice cooling, followed by extraction with ethyl acetate. The organic layer was washed with saturated saline and dried over magnesium sulfate. The solvent was removed under reduced pressure. The residue was purified by flash column chromatography on silica gel (230 g, methylene chloride : ethyl acetate = 20 : 1) to give 4.91 g (95.8%) of methyl [4-(2-azido-1-hydroxyethyl)phenyl]oxyacetate.

$^1$H-NMR ($CDCl_3$) δ: 2.32 (1H, brd, J = 3.2 Hz), 3.40 (1H, dd, J = 4.2, 12.7 Hz), 3.47 (1H, dd, J = 8.0, 12.7 Hz), 3.81 (3H, s), 4.64 (2H, s), 4.81-4.87 (1H, m), 6.91 (2H, d, J = 8.8 Hz), 7.31 (2H, d, J = 8.8 Hz)
FABMS (m/z): 251 ($M^+$)

(e) The compound (2.35 g) prepared in the above step (d) was dissolved in methylene chloride (10 ml). Thionyl chloride (750 μl) was added to the solution under ice cooling. The mixture was stirred at room temperature for one hr. The solvent was removed under reduced pressure to give 2.42 g (96.0%) of methyl [4-(2-azido-1-chloroethyl)phenyl]oxyacetate.

$^1$H-NMR ($CDCl_3$) δ: 3.66 (1H, dd, J = 6.3, 12.9 Hz), 3.73 (1H, dd, J = 7.6, 12.9 Hz), 3.81 (3H, s), 4.65 (2H, s), 4.94 (1H, t, J = 6.8 Hz), 6.91 (2H, d, J = 8.8 Hz), 7.34 (2H, d, J = 8.8 Hz)
FABMS (m/z): 269, 271 ($M^+$ + 1, $M^+$ + 3)

(f) The compound (945 mg) prepared in the above step (e) was dissolved in toluene (8.5 ml). Imidazole (2.38 g) was added to the solution, and the mixture was stirred at 70°C for 22 hr. Water was added to the reaction solution, followed by extraction with ethyl acetate. The organic layer was washed with water and saturated saline and dried over sodium sulfate. The solvent was removed under reduced pressure. The residue was purified by flash column chromatography on silica gel (40 g, methylene chloride : methanol = 50 : 1) to give 641 mg (60.8%) of methyl [4-[2-azido-1-(imidazol-1-yl)ethyl]phenyl]oxyacetate.

$^1$H-NMR ($CDCl_3$) δ: 3.81 (3H, s), 3.90-4.00 (2H, m), 4.64 (2H, s, ), 5.27-5.31 (1H, m), 6.91 (2H, d, J = 8.8 Hz), 6.97 (1H, s), 7.12 (1H, s), 7.15 (2H, d, J = 8.8 Hz), 7.66 (1H, s)
TSPMS (m/z): 302 ($M^+$ + 1)

(g) The compound (80 mg) prepared in the above step (f) was dissolved in methanol (2 ml). 10% palladium-carbon (10 mg) was added to the solution. The mixture was stirred in a hydrogen atmosphere under atmospheric pressure for 1.5 hr. The reaction solution was filtered, and the filtrate was concentrated to give 62 mg (84.7%) of methyl [4-[2-amino-1-(imidazol-1-yl)ethyl]phenyl]oxyacetate.

$^1$H-NMR ($D_2O$) δ: 1.72 (2H, brs), 3.34-3.47 (2H, m), 3.81 (3H, s), 4.63 (2H, s), 5.07-5.15 (1H, m), 6.89 (2H, d, J = 8.8 Hz), 6.99 (1H, s), 7.12 (1H, s), 7.40 (2H, d, J = 8.8 Hz), 7.62 (1H, s)
TSPMS (m/z): 276 ($M^+$ + 1)

(h) 5-t-Butoxycarbonyl-4,5,6,7-tetrahydrothieno[3,2-c]pyridine-2-carboxylic acid (58 mg) was dissolved in dimethylformamide (0.5 ml). BOP (99 mg) and triethylamine (60 μl) were added to the solution under ice cooling. The mixture was stirred for 0.5 hr. A solution of the compound (56 mg), prepared in the above step (g), in dimethylformamide (0.5 ml) was added thereto at the same temperature. The mixture was stirred for 21 hr while raising the temperature to room temperature. Ethyl acetate and a 10% aqueous potassium carbonate solution were added to the reaction solution under ice cooling. The organic layer was separated and washed with saturated

saline. The organic layer was dried over sodium sulfate. The solvent was removed under reduced pressure. The residue was purified by column chromatography on silica gel (6 g, methylene chloride : methanol = 40 : 1 to 30 : 1) to give 92 mg (83.8%) of methyl[4-[1-(imidazol-1-yl)-2-[[5-(t-butoxycarbonyl)-4,5,6,7-tetrahydrothieno[3,2-c]pyridin-2-yl]carbonylamino]ethyl]phenyl]oxyacetate.

> $^1$H-NMR ($CDCl_3$) δ: 1.48 (9H, s), 2.83 (2H, brs), 3.70 (2H, brs), 3.81 (3H, s), 3.80-3.89 (1H, m), 4.21-4.30 (1H, m), 4.42 (2H, brs), 4.64 (2H, s), 5.52 (1H, dd, J = 5.1, 9.5 Hz), 6.91 (2H, d, J = 8.8 Hz), 6.98 (1H, s), 7.10 (1H, s), 7.11 (1H, s), 7.19 (2H, d, J = 8.8 Hz), 7.56 (1H, s)
> TSPMS (m/z): 541 ($M^+$ + 1)

(i) The compound (87 mg) prepared in the above step (h) was dissolved in methanol(1.6 ml). A 1 N aqueous sodium hydroxide solution (320 μl) was added to the solution at room temperature, and the mixture was stirred for one hr. The reaction solution was concentrated. Water was added to the concentrate. The mixture was washed with ether. The aqueous layer was adjusted to pH = 4 by the addition of acetic acid and saturated with salt, followed by extraction with n-butanol. The organic layer was dried over sodium sulfate. The solvent wag removed under reduced pressure. Water was added to the residue, and the resultant precipitate was collected by filtration to give 43.5 mg (51.3%) of [4-[1-(imidazol-1-yl)-2-[[5-(t-butoxycarbonyl)-4,5,6,7-tetrahydrothieno[3,2-c]pyridin-2-yl]carbonylamino]ethyl]phenyl]oxyacetic acid.

> $^1$H-NMR ($CD_3OD$) δ: 1.47 (9H, s), 2.83 (2H, brt, J = 5.4 Hz), 3.70 (2H, brt, J = 5.4 Hz), 3.97 (1H, dd, J = 5.6, 14.1 Hz), 4.20 (1H, dd, J = 9.5, 14.1 Hz), 4.43 (2H, brs), 4.55 (2H, s), 5.67 (1H, dd, J = 5.6, 14.1 Hz), 6.97 (2H, d, J = 8.8 Hz), 7.25 (1H, brs), 7.27 (1H, s), 7.36 (2H, d, J = 8.8 Hz), 7.45 (1H, brs), 8.42 (1H, brs)
> FABMS (m/z): 527 ($M^+$ + 1)

(j) The compound (40 mg) prepared in the above step (i) was suspended in methylene chloride (1 ml). Anisole (40 μl) and trifluoroacetic acid (170 μl) were added to the solution under ice cooling. The mixture was stirred at room temperature for one hr. The reaction solution was concentrated. Water was added to the concentrate. The mixture was washed with diisopropyl ether and ether. The aqueous layer was lyophilized to give 42 mg (100%) of the title compound.

> $^1$H-NMR ($CD_3OD$) δ: 3.16 (2H, t, J = 6.1 Hz), 3.54 (2H, t, J = 6.1 Hz), 4.02 (1H, dd, J = 5.6, 14.1 Hz), 4.26 (2H, s), 4.29 (1H, dd, J = 9.5, 14.1 Hz), 4.64 (2H, s), 5.74-5.80 (1H, m), 7.00 (2H, d, J = 8.8 Hz), 7.37 (1H, s), 7.42 (2H, d, J = 8.8 Hz), 7.45 (1H, brs), 7.64 (1H, brs), 8.91 (1H, brs)
> FABMS (m/z): 427 ($M^+$ + 1)

Example 14

[4-[1-(Imidazol-1-yl)-2-[4-(piperidin-4-yl)-2-oxopiperazin-1-yl]ethyl]phenyl]oxyacetic acid trifluoroacetate

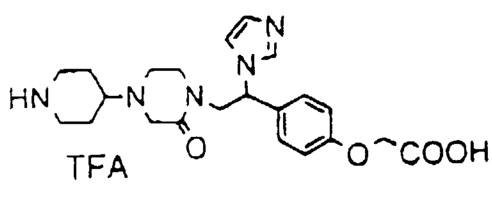

(a) t-Butyl [4-[[4-(piperidin-4-yl)-2-oxopiperazin-1-yl]acetyl]phenyl]oxyacetate (1.06 g, WO 9602503) was dissolved in methanol (10 ml). Sodium borohydride (30.3 mg) was added to the solution under ice cooling. The mixture was stirred at room temperature for 2 hr. Further, sodium borohydride (37.8 mg) was added thereto under ice cooling, and the mixture was stirred at room temperature for 1.5 hr. Water was added to the reaction solution. Methanol was removed under reduced pressure, followed by extraction with chloroform. The organic layer was washed with saturated saline and dried over magnesium sulfate. The solvent was removed under reduced pressure. The residue was purified by column chromatography on silica gel (30 g, chloroform : methanol = 70 : 1) to give 823 mg (77.1%) of t-butyl [4-[1-hydroxy-2-[4-(piperidin-4-yl)-2-oxopiperazin-1-yl]ethyl]phenyl]oxyacetate.

$^1$H-NMR ($CDCl_3$) δ: 1.31-1.44 (2H, m), 1.46 (9H, s), 1.49 (9H, s), 1.77 (2H, brd, J = 13.6 Hz), 2.35-2.44 (1H, m), 2.53-2.80 (4H, m), 3.00-3.09 (1H, m), 3.21-3.37 (3H, m), 3.51-3.63 (2H, m), 4.10 (2H, brs), 4.42 (1H, d, J = 3.6 Hz), 4.51 (2H, s), 4.94-5.01 (1H, m), 6.88 (2H, d, J = 8.6 Hz), 7.30 (2H, d, J = 8.6 Hz)
FDMS (m/z): 533 ($M^+$)

(b) The compound (492 mg) prepared in the above step (a) was dissolved in methylene chloride (2 ml). Thionyl chloride (70 μl) was added to the solution under ice cooling. The mixture was stirred at room temperature for 15 min and further stirred at 40 to 45°C for 1 hr. The solvent was removed under reduced pressure. Toluene (1.5 ml) and imidazole (188 mg) were added to the residue. The mixture was heated under reflux with stirring for 5 hr. To the reaction solution was added 0.5 N hydrochloric acid. The mixture was washed with ether. The aqueous layer was rendered alkaline by the addition of potassium carbonate, followed by extraction with methylene chloride. The organic layer was washed with saturated saline and dried over sodium sulfate. The solvent was removed under reduced pressure. The residue was purified by column chromatography on silica gel (17 g, methylene chloride : methanol = 30 : 1 to 15 : 1) to give 98 mg (18.3%) of t-butyl [4-[1-(imidazol-1-yl)-2-[4-(piperidin-4-yl)-2-oxopiperazin-1-yl]ethyl]phenyl]oxyacetate.

$^1$H-NMR ($CDCl_3$) δ: 1.26-1.40 (2H, m), 1.45 (9H, s), 1.48 (9H, s), 1.73 (2H, brd, J = 11.9 Hz), 2.31-2.40 (1H, m), 2.42-2.50 (1H, m), 2.51-2.64 (2H, m), 2.71 (2H, brt, J = 12.2), 3.01-3.09 (1H, m), 3.25 (2H, q, J = 16.3 Hz), 3.65 (1H, dd, J = 10.0, 13.6 Hz), 4.10 (2H, brs), 4.21 (1H, dd, J = 4.9, 13.6 Hz), 4.51 (2H, s), 5.60 (1H, dd, J = 4.9, 10.0 Hz), 6.89 (2H, d, J = 8.8 Hz), 6.96 (1H, s), 7.08 (1H, s), 7.20 (2H, d, J = 8.8 Hz), 7.56 (1H, s)
TSPMS (m/z): 541 ($M^+$ + 1)

(c) The compound (95 mg) prepared in the above step (b) was dissolved in methylene chloride (1.6 ml). Anisole (35 μl) and trifluoroacetic acid (130 μl) were added to the solution under ice cooling. After 1.5 hr, anisole (35 μl) and trifluoroacetic acid (130 μl) were added thereto, and, in addition, one hr after that, trifluoroacetic acid (130 μl) was added thereto. The mixture was stirred at room temperature for 4.5 hr. The reaction solution was concentrated. Water was added to the concentrate. The mixture was washed with diisopropyl ether and ether. The aqueous layer was lyophilized to give 85 mg (79.8%) of the title compound.

$^1$H-NMR ($CDCl_3$) δ: 1.66-1.80 (2H, m), 2.21 (2H, brd, J = 13.6 Hz), 2.96 (2H, brd, J = 12.4 Hz), 3.16-3.35 (3H, m), 3.38-3.53 (4H, m), 3.70 (2H, s), 3.99 (1H, dd, J = 7.3, 14.1 Hz), 4.49 (1H, dd, J = 8.3, 14.1 Hz), 4.58 (2H, s), 5.80 (1H, t, J = 7.8 Hz), 6.95 (2H, d, J = 8.8 Hz), 6.92-7.00 (3H, m), 7.53 (1H, s), 8.74 (1H, s)
FABMS (m/z): 428 ($M^+$ + 1)

Test 1

Inhibitory activity against platelet aggregation

The inhibitory activity of the compound according to the present invention against platelet aggregation was examined with human PRP (platelet rich plasma).

Nine volumes of a blood sample was taken out of the vein of a normal male human being with a syringe in which one volume of a 3.8% sodium citrate solution was charged. The blood sample was centrifuged at 170 x g at room temperature for 10 min. The supernatant thus obtained was isolated as PRP. The residual blood sample that PRP had been taken out was centrifuged at 2,700 x g for 15 min. The supernatant was then taken as platelet poor plasma (PPP).

A platelet aggregation test was carried out with an aggligometer (PAM-8C, manufactured by MEBANICKS Co. Ltd.). Compounds under test were dissolved in physiological saline, 0.1 N hydrochloric acid-physiological saline, or 1 N aqueous sodium hydroxide solution-physiological saline. The compound under test and PRP were preincubated for 2 min. An aggregation inducer ADP (CHRONO-PAR REAGENTS 384 ADP, CHRONO-LOG Corp.) was used after dilution with physiological saline to a final concentration of 5 μM.

The anti-platelet aggregation activity was determined as an inhibition rate to platelet aggregation effect of ADP in the absence of a compound under tent as follows.

Anti-platelet aggregation activity (%) = [1 - (aggregation rate by ADP in the presence of test compound/aggregation rate by ADP in the absence of test compound)] x 100

The inhibitory activity of the compounds of the present invention against platelet aggregation was as follows.

| Compound No. of Ex. | $IC_{50}$ (μM) |
|---|---|
| 1 | 4.4 |
| 2 | 9.3 |
| 3 | 0.36 |
| 4 | 0.14 |
| 5 | 0.28 |
| 6 | 0.071 |
| 7 | 0.093 |
| 8 | 0.031 |

Test 2

Inhibitory activity against binding between platelet GPIIb/IIIa and fibrinogen

The inhibitory activity of the compounds of the present invention against binding between platelet GPIIb/IIIa and fibrinogen was examined by means of a human platelet GPIIb/IIIa solid phase receptor binding experiment system using a biotinylated human fibrinogen as a ligand.

At the outset, human platelet GPIIb/IIIa was purified according to a method established by Pytela, R et al. (Science, 231, 1559-1561 (1986)). Specifically, platelets collected from a normal human being was washed with TBS (0.15 M NaCl/50 mM Tris-HCl buffer (pH 7.5)) containing 0.2% (w/v) glucose. To the resultant pellet was added an equal amount of TBS containing 50 mM octylglucoside and 2 mM PMSF, followed by solubilization of membrane protein at 4°C for 20 min. The extract was centrifuged under conditions of 4°C, 3500 x g, and 20 min. To the supernatant were added 1 mM $CaCl_2$ and 1 mM $MgCl_2$. Thus, a sample for affinity chromatography was prepared. The solubilized protein solution was adsorbed on a GRGDSPK-Sepharose column which had been previously equilibrated with TBS containing 50 mM octylglucoside, 1 mM $CaCl_2$, and 1 mM $MgCl_2$ (buffer A). After washing with buffer A, the adsorbed GPIIb/IIIa was eluted with buffer A containing 2 mM GRGDSPK peptide.

The GRGDSPK-Sepharose was prepared by coupling of GRGDSPK peptide with CNBr-activated Sepharose 4B (manufactured by Pharmacia) according to the instruction manual of the manufacturer.

Next, a human platelet GPIIb/IIIa solid phase receptor binding test was carried out according to a method established by Mori et al. (Nippon Kessen Shiketsu Gakkaishi, 2(4), 323-329 (1991)).

Purified human platelet GPIIb/IIIa was adjusted to 2 μg/ml, and an 50 μl aliquot was adsorbed on each well of a 96 well microtiter plate at 4°C overnight. After washing with TBS containing 1 mM $CaCl_2$ and 1 mM $MgCl_2$, 100 μl of 1% bovine serum albumin was added to each of the wells, followed by blocking at 4°C overnight. The microtiter plate was then washed with TBS containing 0.01% Tween 20 (TBS-Tween 20), and 50 μl of biotinylated fibrinogen, which had been adjusted to 1 μg/ml, was added to each of the wells. Simultaneously, 50 μl of the test compound, which had been adjusted to appropriate concentrations, was added thereto, followed by a reaction at room temperature for 4 hr. After washing with TBS-Tween 20, 50 μl of peroxidase-labelled avidin, which had been diluted 4000 times with TBS, was added to each of the wells, followed by a reaction for 20 min. After washing with TBS-Tween 20, 50 μl of a solution of 1 mg/ml ABTS (2,2'-azino-bis(3-ethylbenzthiazoline-6-sulfonic acid) dissolved in a peroxidase substrate buffer, which had been diluted 10 times, was added to each of the wells, and a reaction was allowed to proceed for 5 min. Next, 50 μl of a 0.1 M citric acid buffer (pH 4.3) containing 0.05% $NaN_3$ was added to each of the wells to terminate the reaction, and the absorbance was measured at 415 nm.

The binding inhibition was calculated by the following equation.

Binding inhibition (%) = [1 - (absorbance with test compound/absorbance without test compound)] x 100

The inhibitory activity of the compounds of the present invention against binding between platelet GPIIb/IIIa and fibrinogen was as follows.

| Compound No. of Ex. | $IC_{50}$ (μM) |
|---|---|
| 1 | 0.27 |
| 2 | 0.27 |
| 3 | 0.0014 |
| 4 | 0.0048 |
| 5 | 0.01 |
| 6 | 0.012 |
| 7 | 0.023 |
| 8 | 0.19 |

Test 3

Inhibitory activity against thromboxane $A_2$ ($TXA_2$) synthetase

The inhibitory activity of the compound of the present invention against $TXA_2$ synthetase was assayed according to a method established by Ri et al. (Nihon Yakuri Gakkaishi, 106, 31 (1995)). A specimen solution (10 μl) was added to 100 μl of a 50 mM Tris-HCl buffer (containing 0.1 M NaCl, pH 7.5, 25°C) containing 10 μg of human $TXA_2$ synthetase (BIOMOL), and incubation was then carried out at 25°C for 3 min. Next, 2 μl of 50 μg/ml $PGH_2$ solution (Cayman Chemical) was added thereto to initiate an enzyme reaction, followed by the reaction at 25°C for 3 min. An aqueous ferrous chloride solution (25 mM, 10 μl) was added thereto to terminate the reaction. The reaction system was allowed to stand at room temperature for 15 min and then centrifuged under conditions of 4°C, 3000 x g, and 10 min. The supernatant was obtained as a sample for quantitative determination of $TXB_2$. The amount of $TXB_2$ was determined by means of Thromboxane $B_2$ Enzyme Immunoassay Kit (Cayman Chemical). After a color reaction, the absorbance was measured at 415 nm with a microplate reader (MTP-32, manufactured by Corona Electric Co., Ltd.). The inhibition against $TXA_2$ synthetase activity was calculated by the following equation, and 50% inhibition concentration was determined by regression straight line.

$$\text{Inhibition (\%)} = (1 - B/A) \times 100$$

wherein

A represents the amount of $TXB_2$ produced without the test compound; and
B represents the amount of $TXB_2$ produced with the test compound.

The inhibitory activity of the compounds of the present invention against $TXA_2$ synthetase was as follows.

| Compound No. of Ex. | $IC_{50}$ (μM) |
|---|---|
| 1 | 0.36 |
| 2 | > 10 |
| 3 | 0.55 |
| 4 | 0.87 |
| 5 | 0.16 |
| 6 | 0.57 |
| 7 | > 10 |
| 8 | 0.69 |

## Claims

1. An imidazole or pyridine derivative represented by the following formula (I) or a pharmacologically acceptable salt and a solvate thereof:

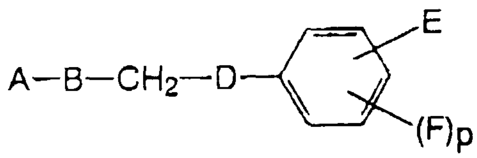

( I )

wherein

A represents the following formula (II), (III), or (IV):

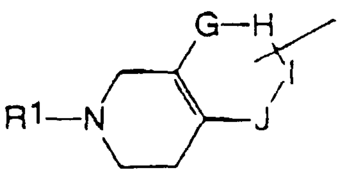

( II )

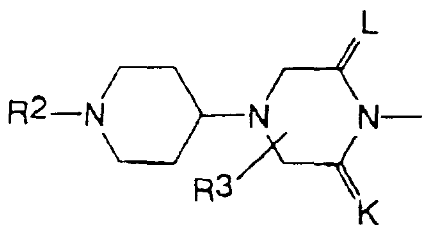

( III )

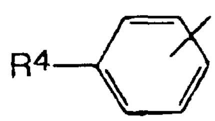

( IV )

wherein

$R^1$ and $R^2$, which may be the same or different, represents
a hydrogen atom or
lower alkyl in which one or more hydrogen atoms are optionally substituted by a hydroxyl group, a halogen atom, amino, or lower alkylamino;
$R^3$ represents
a hydrogen atom,
lower alkyl in which one or more hydrogen atoms are optionally substituted by a hydroxyl group, a halogen atom, amino, carboxyl, lower alkoxy, lower alkylamino, or lower alkoxycarbonyl,
phenyl in which one or more hydrogen atoms are optionally substituted by a hydroxyl group, a halogen atom, amino, carboxyl, lower alkoxy, lower alkylamino, lower alkoxycarbonyl, or halo lower alkyl, or
phenyl lower alkyl in which one or more hydrogen atoms are optionally substituted by a hydroxyl group, a halogen atom, amino, carboxyl, lower alkoxy, lower alkylamino, lower alkoxycarbonyl, or halo lower alkyl,
$R^4$ represents amidino or aminomethyl,
G, H, I, and J, which may be the same or different, represent $-CR^5=$, $-CR^5R^6-$, -N=, $-NR^5-$, -O-, -S-, -(CO)-, or a bond wherein $R^5$ and $R^6$, which may be the same or different, represent a hydrogen atom or lower alkyl, and
K and L represent $H_2$ or an oxygen atom;

B represents $-COCHR^7-$, $-CONR^7-$ wherein $R^7$ represents a hydrogen atom or lower alkyl, or a bond;
D represents -(CO)-, -CHOH-, or -CHY- wherein Y represents pyridyloxy, pyridyl, pyridyl lower alkyl, imidazolyl, or imidazolyl lower alkyl;
E represents a hydrogen atom (provided that E does not represent a hydrogen atom when D represents -(CO)-) or $-CH_2Y$ wherein Y is as defined above;
F represents group $-Z-(CH_2)_qCOOR^8$ or $-CH=CR^9COOR^8$ wherein

Z represents -O- or a bond,

$R^8$ represents a hydrogen atom, lower alkyl, or an ester residue removable under physiological conditions,
$R^9$ represents a hydrogen atom or lower alkyl, and
q is an integer of 1 to 4; and

p is 1 or 2.

2. The compound according to claim 1, wherein A represents the formula (II).

3. The compound according to claim 2, wherein A represents the formula (II) wherein any one of G and J represents $-NR^5-$, -O-, or -S- with the other representing a bond and both H and I represent $-CR^5=$.

4. The compound according to claim 1, wherein A represents the formula (III).

5. The compound according to claim 4, wherein A represents the formula (III) wherein any one of or both K and L represent an oxygen atom.

6. The compound according to claim 1, wherein A represents the formula (IV).

7. The compound according to claim 1, wherein D represents -CHY- wherein Y represents 1-imidazolyl.

8. The compound according to claim 1, wherein D represents -CHY- wherein Y represents 3-pyridyl.

9. The compound according to claim 1, wherein D represents -CHY- wherein Y represents 3-pyridyloxy.

10. The compound according to claim 1, wherein E represents $-CH_2Y$ wherein Y represents 1-imidazolyl.

11. The compound according to claim 1, wherein A represents the formula (II) and B represents $-CONR^7-$.

12. The compound according to claim 11, wherein $R^7$ represents a hydrogen atom or methyl.

13. The compound according to claim 11 or 12, wherein D represents -(CO)- or -CHY-.

14. The compound according to claim 13, wherein Y represents pyridyloxy, pyridyl, pyridylmethyl, or imidazolyl.

15. The compound according to claim 1, wherein A represents the formula (III) and B represents a bond.

16. The compound according to claim 15, wherein D represents -(CO)- or -CHY-.

17. The compound according to claim 1, which is selected from

[2-(imidazol-1-yl)methyl-4-[[[4,5,6,7-tetrahydrothieno[3,2-c]pyridin-2-yl]carbonylamino]acetyl]phenyl]oxyacetic acid,
2-(pyridin-3-yl)oxymethyl-4-[[[4,5,6,7-tetrahydrothieno[3,2-c]pyridin-2- yl]carbonylamino]acetyl]phenyloxyacetic acid,
[[4-[2-[[4,5,6,7-tetrahydrothieno[3,2-c]pyridin-2-yl]carbonyl]amino-1-[(pyridin-3-yl)oxy]ethyl]]-o-phenylene]dioxy]diacetic acid,
[[4-[2-[[(4,5,6,7-tetrahydrothieno[3,2-c]pyridin-2-yl)carbonyl]-N-methylamino]-1-[(pyridin-3 yl)oxy]ethyl]-o-phenylene]dioxy]diacetic acid,
[4-[2-[[(4,5,6,7-tetrahydrothieno[3,2-c]pyridin-2-yl)carbonyl]amino-1-[(pyridin-3-yl)oxy]ethyl]phenyl]oxyacetic acid,
[2-(1-imidazolylmethyl)-4-[[4-(piperidin-4-yl)-2-oxopiperazin-1-yl]acetyl]phenyl]oxyacetic acid,
[4-[[4-(piperidin-4-yl)-2-oxopiperazin-1-yl]acetyl]-2-(3-pyridyloxymethyl)phenyl]oxyacetic acid,
[2-(1-imidazolylmethyl)-4-[[4-(piperidin-4-yl)-2, 6-dioxopiperazin-1-yl]acetyl]phenyl]oxyacetic acid,
ethyl [4-[1-(pyridin-3-yl)oxy-2-[[(4,5,6,7-tetrahydrothieno[3,2-c]pyridin-2-yl)carbonyl]amino]ethyl]phenyl]oxyacetate,
[4-[2-[[(4,5,6,7-tetrahydrothieno[3,2-c]pyridin-2-yl]carbonyl]amino]-1-(pyridin-3-yl)ethyl]phenyl]oxyacetic acid,
[4-[2-[[(4,5,6,7-tetrahydrothieno[3,2-c]pyridin-2-yl)carbonyl)-N-methylamino)-1-(pyridin-3-yl)oxyethyl]phenyl]oxyacetic acid,

[4-[1-(pyridyl-3-ylmethyl)-2-[[(4,5,6,7-tetrahydrothieno[3,2-c]pyridin-2-yl)carbonyl]amino]ethyl]phenyl]oxyacetic acid,
[4-[1-(imidazol-1-yl)-2-[[(4,5,6,7-tetrahydrothieno[3,2-c]pyridin-2-yl)carbonyl]amino]ethyl]phenyl]oxyacetic acid, and
[4-[1-(imidazol-1-y1)-2-[4-(piperidin-4-yl)-2-oxopiperazin-1-yl)ethyl]phenyl]oxyacetic acid.

**18.** A pharmaceutical composition comprising an effective amount of the compound according to any one of claims 1 to 17 or a pharmacologically acceptable salt or solvate thereof and a pharmacologically acceptable carrier.

**19.** The pharmaceutical composition according to claim 18, which is used as the treatment or prevention of thrombotic diseases.

**20.** Use of the compound according to any one of claims 1 to 17 for the treatment or prevention of thrombotic diseases.

**21.** Use of the compound according to any one of claims 1 to 17 for the production of a therapeutic or prophylactic agent for thrombotic diseases.

**22.** A method for treating or preventing thrombotic diseases, comprising the step of administering an effective amount of the compound according to any one of claims 1 to 14 or a pharmacologically acceptable salt or solvate thereof to mammals including human beings.

## INTERNATIONAL SEARCH REPORT

International application No.
PCT/JP97/01106

A. CLASSIFICATION OF SUBJECT MATTER

Int. $Cl^6$ C07D401/14, 495/04 // A61K31/445, 495

According to International Patent Classification (IPC) or to both national classification and IPC

B. FIELDS SEARCHED

Minimum documentation searched (classification system followed by classification symbols)

Int. $Cl^6$ C07D401/14, 495/04 // A61K31/445, 495

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| A | JP, 6-506200, A (Pfizer Inc.), July 14, 1994 (14. 07. 94), Claim & WO, 92/17451, A & PT, 100332, A & FI, 9304045, A & EP, 579618, A & US, 5457118, A | 1-19, 21 |
| A | WO, 94/26745, A (Merck & Co., Inc.), November 24, 1994 (24. 11. 94), Claim & JP, 8-509982, A & EP, 698023, A & US, 5334596, A & AU, 9468221, A | 1-19, 21 |
| P,A | JP, 8-113563, A (Meiji Seika Kaisha, Ltd.), May 7, 1996 (07. 05. 96), Claim (Family: none) | 1-19, 21 |
| P,A | WO, 96/11180, A (Meiji Seika Kaisha, Ltd.), April 18, 1996 (18. 04. 96), Claim & EP, 738705, A | 1-19, 21 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| June 16, 1997 (16. 06. 97) | June 24, 1997 (24. 06. 97) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP97/01106

**Box I Observations where certain claims were found unsearchable (Continuation of item 1 of first sheet)**

This international search report has not been established in respect of certain claims under Article 17(2)(a) for the following reasons:

1. [X] Claims Nos.: 20, 22
because they relate to subject matter not required to be searched by this Authority, namely:
The inventions related to claims 20 and 22 fall under the category of methods for treatment of the human or animal body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1992)